# EUROPEAN PATENT SPECIFICATION

(11) **EP 4 138 732 B1**
(45) Date of publication and mention of the grant of the patent: **18.03.2026**
(21) Application number: 21740304.7
(22) Date of filing: 14.06.2021
(51) Int. Cl.: A61F 2/24, A61F 2/962

(54) **NOSE CONE FOR DELIVERY SYSTEMS**
NASENKONUS FÜR FREISETZUNGSSYSTEME
CÔNE DE NEZ POUR SYSTÈMES DE DISTRIBUTION

(30) Priority: 15.06.2020 US 202063039028 P
(43) Date of publication of application: 01.03.2023
(73) Proprietor: Edwards Lifesciences Corporation, Irvine, CA 92614-5688 (US)
(72) Inventor: LEIBA, Eyal, 32022 N. Misgav (IL); GOLDBERG, Eran, 30889 Caesarea (IL)
(74) Representative: Eisenführ Speiser
(86) International application number: PCT/US2021/037242
(87) International publication number: WO 2021/257459

(56) References cited:
- WO-A1-2015/127283
- WO-A1-2017/035381
- WO-A1-2017/096166
- WO-A2-2012/116368
- US-A- 5 327 906
- US-A1- 2015 073 339

## Description

### RELATED APPLICATION

This application claims the benefit of and priority to U.S. Provisional Patent Application No. 63/039,028 filed on June 15, 2020.

### BACKGROUND OF THE INVENTION

A variety of maladies may affect an individual's body. Such maladies may be of the individual's heart, and may include maladies of the individual's heart valves, including the aortic, mitral, tricuspid, and pulmonary valves. Stenosis, for example, is a common and serious valve disease that may affect the operation of the heart valves and an individual's overall well-being.

Implants may be provided that may replace or repair portions of a patient's heart. Prosthetic implants, such as prosthetic heart valves, may be provided to replace a portion of a patient's heart. Prosthetic aortic, mitral, tricuspid, and even pulmonary valves may be provided.

Implants may be deployed to the desired portion of the patient's body percutaneously, in a minimally invasive manner. Such deployment may occur transcatheter, in which a catheter may be deployed through the vasculature of an individual.

Devices for delivery of the implants may be complex and may involve assembly of many intricate parts. As such, flexibility in assembly of these complex delivery devices may be beneficial.

WO 2017/035381 A1 describes devices, systems and methods for implantation of a prosthesis within a lumen or body cavity and delivery systems for delivering the prosthesis to a location for implantation. A delivery system can include a tether connected to a single directional handle knob for release of the prosthesis within the lumen or body cavity and retraction of the tether towards the handle.

### SUMMARY

The invention relates to a delivery device as defined by claim 1. The present devices, systems, and methods may relate to delivery systems and may relate to nose cones for such delivery systems. In one embodiment, a delivery system for an implant may be provided. The delivery system may include an elongate shaft having a distal end and a proximal end and including an implant retention area for retaining the implant. The delivery system may include a nose cone. The delivery system may include one or more angled projections configured to couple the nose cone to the distal end of the elongate shaft.

In one embodiment, a delivery system for an implant may be provided. The delivery system may include an elongate shaft having a distal end and a proximal end and including an implant retention area for retaining the implant. The delivery system may include a nose cone. The delivery system may include a threaded coupler configured to couple the nose cone to the distal end of the elongate shaft.

In one embodiment, a method may be provided. The method may be of assembling at least a portion of a delivery system for an implant. The method may include coupling a nose cone to a distal end of an elongate shaft of the delivery system by translating a proximal end of the nose cone relative to the distal end of the elongate shaft, the elongate shaft including an implant retention area for retaining the implant.

In one embodiment, a nose cone for a delivery system for an implant may be provided. The nose cone may include a proximal opening at a proximal end, the proximal opening configured to receive a distal end of an elongate shaft of the delivery system. The nose cone may include an inner surface defining a lumen, the inner surface configured to engage or having one or more angled projections configured to couple the nose cone to the distal end of the elongate shaft.

In one embodiment, a nose cone for a delivery system for an implant may be provided. The nose cone may include a proximal opening at a proximal end, the proximal opening configured to receive a distal end of an elongate shaft of the delivery system. The nose cone may include an inner surface defining a lumen. The nose cone may include nose cone threads located within the lumen and configured to engage a first set of threads coupled to the elongate shaft of the delivery system to couple the nose cone to the distal end of the elongate shaft.

### BRIEF DESCRIPTION OF THE DRAWINGS

These and other features, aspects, and advantages are described below with reference to the drawings, which are intended to illustrate, but not to limit, the disclosure. In the drawings, like reference characters denote corresponding features consistently throughout similar embodiments.
FIG. 1 is a side view of a delivery apparatus according to an embodiment of the present disclosure.
FIG. 2A illustrates a side view of an implant according to an embodiment of the present disclosure.
FIG. 2B illustrates a perspective view of an implant according to an embodiment of the present disclosure.
FIG. 2C illustrates a perspective view of an implant according to an embodiment of the present disclosure.
FIG. 2D illustrates a perspective view of the implant shown in FIG. 2C with an outer covering removed according to an embodiment of the present disclosure.
FIG. 2E illustrates a perspective view of a frame of the implant shown in FIG. 2C in a compressed state according to an embodiment of the present disclosure.
FIGS. 3A-3B are views of components of the delivery apparatus shown in FIG. 1.
FIGS. 4A-4D are side cross sectional views of a nose cone and elongate shaft according to an embodiment of the present disclosure.
FIGS. 5A-5D are side cross sectional views of a nose cone and elongate shaft according to an embodiment of the present disclosure.
FIGS. 6A-6C are side cross sectional views of a nose cone and elongate shaft according to an embodiment of the present disclosure.
FIGS. 7A-7D are side cross sectional views of a nose cone and elongate shaft according to an embodiment of the present disclosure.
FIGS. 8A-8C are side cross sectional views of a nose cone and elongate shaft according to an embodiment of the present disclosure.
FIGS. 9A-9C are side cross sectional views of a nose cone and elongate shaft according to an embodiment of the present disclosure.
FIG. 10 is a flowchart of a method according to an embodiment of the present disclosure.

### DETAILED DESCRIPTION

The following description and examples illustrate some example embodiments of the disclosure in detail. Those of skill in the art will recognize that there are numerous variations and modifications of the disclosure that are encompassed by its scope. Accordingly, the description of a certain example embodiment should not be deemed to limit the scope of the present disclosure.

Delivery systems for implants in the form of prosthetic valves may include a distal nose cone rigidly attached to a nose cone shaft. The nose cone shaft may include an internal lumen sized to accommodate a guidewire extending therethrough. Conventional nose cones may be fixedly attached using a process that requires the attachment of the nose cone to the nose cone shaft prior to assembly of the remaining components of the delivery system. This nose cone attachment process limits the sequence of assembling a delivery system, as the distal end of the nose cone shaft is occupied by the nose cone, so the remaining delivery system components are assembled from a proximal end of the nose cone shaft (e.g., near the handle of the delivery system). This constraint complicates the delivery system assembly process. In addition, the presence of an attached nose cone may interfere with the process of crimping an implant. Thus, it may be desirable to provide a mode of attachment between the nose cone and the nose cone shaft that may obviate this assembly-sequence limitation.

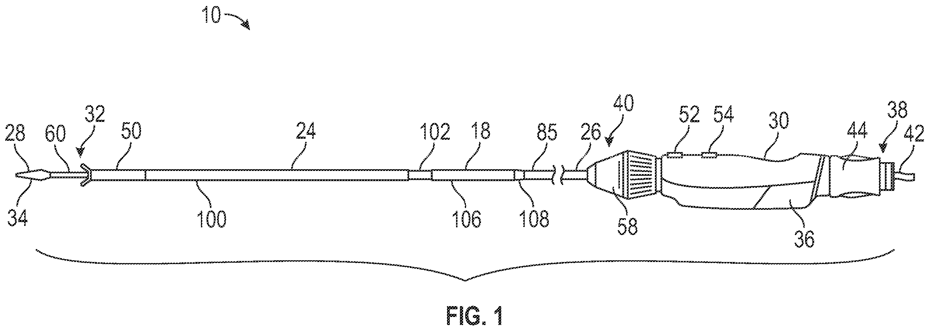

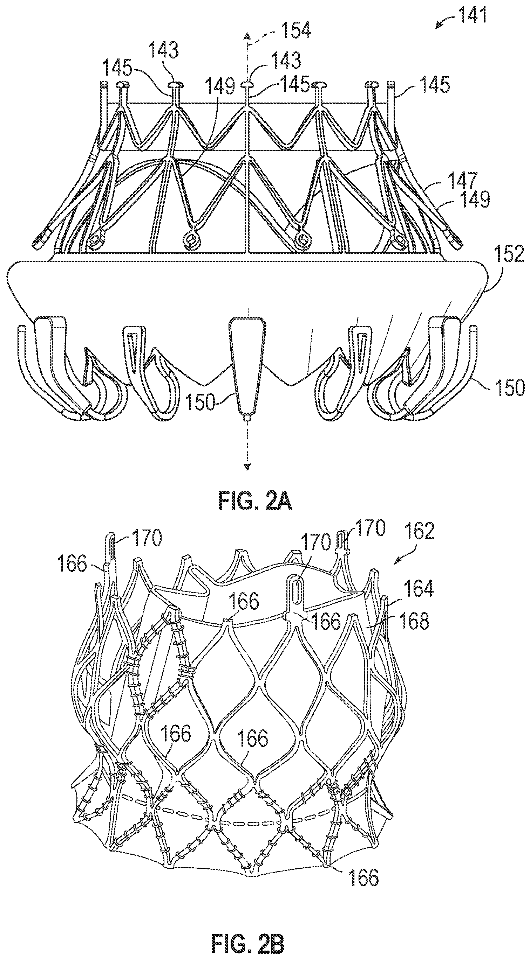

FIG. 1 illustrates an embodiment of a delivery system 10 for an implant, which may be similar to the implants shown in FIGS. 2A-2E. The implant may be a mitral, tricuspid, or pulmonary prosthetic valve, among other forms of prosthetics. The implant may comprise a stent, clip, or other form of implant that may be inserted in a portion of the patient's body, including the patient's heart.

FIG. 2A, for example, illustrates an implant 141 including couplers 143 in the form of tabs at a proximal end of the implant 141. The couplers 143 may be positioned at the ends of struts 145 of a frame 147 of the implant 141. The implant 141 may include proximal anchors 149 and may include distal anchors 150. The anchors may be configured to secure the implant to a native valve location. The implant 141 may comprise a prosthetic replacement mitral heart valve, and the distal anchors 150 may extend over leaflets of the native mitral valve. The proximal anchors 149 may be positioned on the atrial side of the native mitral valve. The implant 141 may include a skirt 152 and may extend around an axis 154.

The implant 141 may comprise a self-expanding implant, and may be configured to expand within a patient's body upon being released from an implant retention area of a delivery apparatus. For example, the self-expanding anchor may have a capsule covering the implant and then the capsule may be retracted from the implant to uncover the self-expanding implant and allow the implant to expand. Such an implant may be made of a nitinol material (e.g., a nitinol frame) or other shape memory material as desired. FIG. 2A illustrates the implant 141 in an expanded or deployed state.

Other forms of implants may include prosthetic replacement aortic valves. FIG. 2B, for example, illustrates an embodiment of a prosthetic replacement aortic valve. The implant 162 may be an expandable implant as shown in FIG. 2B, which may be configured to be expanded to be placed in position within the native valve location. The implant 162 may include a frame 164 including a plurality of supports 166 configured to be compressed for positioning within the delivery apparatus and configured to be expanded at the desired time. The frame 164 may support prosthetic valve leaflets 168 that operate in lieu of the native valve leaflets. The frame 164 may include couplers 170 for coupling to the delivery apparatus, to retain the implant to the delivery apparatus until deployment is desired. The couplers 170 may comprise apertures as shown in FIG. 2B, or may have other forms as desired.

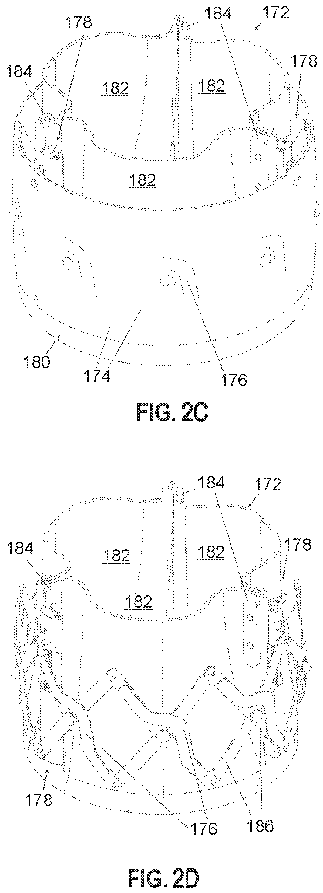

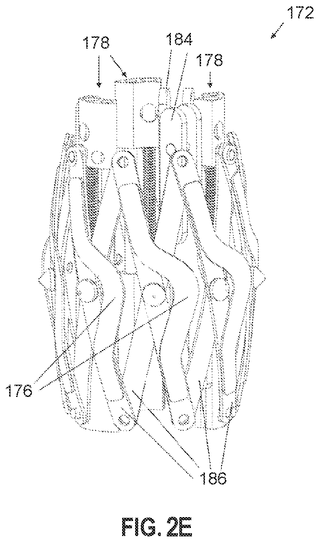

Other forms of implants may include mechanically expandable implants. A mechanically expandable implant may expand due to operation of a mechanical assembly. An example of such an implant is disclosed in U.S. Patent No. 9,913,716, filed January 24, 2017 and issued March 13, 2018, the entire contents of which are incorporated herein. FIGS. 72, 77, and 81 of U.S. Patent No. 9,913,716 are reproduced here as FIGS. 2C-2E. The implant may include a prosthetic replacement heart valve assembly 172, a stent lattice 174, graft enclosures 176, jack assemblies 178, graft material 180, valve leaflets 182, and commissure plates 184. A cover is removed in FIG. 2D to show struts 186. FIG. 2E illustrates the implant with the cover removed, and in a compressed state. Any of the crimping devices disclosed herein may be utilized to move the implant to a compressed state as shown in FIG. 2E. A mechanical assembly may then be utilized to expand the implant at a desired location within the patient's body.

Referring back to FIG. 1, the delivery system 10 may include a delivery apparatus 18 that may include an elongate shaft 24 including a proximal end 26 and a distal end 28 and having a length between the proximal end 26 and the distal end 28. A housing in the form of a handle 30 may be positioned at a proximal portion of the elongate shaft 24 including the proximal end 26 of the elongate shaft 24. The handle 30 may be configured for an individual to grip to utilize when operating the delivery apparatus 18. The elongate shaft 24 may extend outward from the handle 30 and may be configured to be inserted into a patient's body to be directed to a desired treatment site of the patient's body. The elongate shaft 24 may be configured to be inserted into the vasculature of the patient's body, or otherwise may be inserted into the vasculature of the patient's body. Such insertion may be percutaneous and minimally invasive, such as transfemoral entry. Other forms of entry, such as transapical may be utilized as well. The handle 30 may remain exterior to the patient's body during insertion.

The elongate shaft 24 may include an implant retention area 32, which may be covered by a sheath to form a capsule. The implant retention area 32 may be configured to retain the implant. The implant may be retained in the implant retention area 32 until the desired time for deployment of the implant. The elongate shaft 24 may be inserted into the patient's body and navigated to the desired deployment location to position the implant retention area 32 as desired. The delivery apparatus 18 may then be operated to deploy the implant from the implant retention area 32.

The elongate shaft 24 may further include a nose cone 34 at the distal end 28 of the elongate shaft 24. The nose cone 34 may form the tip of the elongate shaft 24 and may be pliable to avoid injury to portions of the patient's body contacted by the tip of the elongate shaft 24. The nose cone 34 may have a tapered shape, from its proximal end to its distal end.

The handle 30 may include an outer surface 36 for being gripped, and may include a proximal portion 38 and a distal portion 40. The outer surface 36 of the handle 30 may be configured to be ergonomic, to be gripped by the user. The handle 30 may be configured to be driven distally to move the elongate shaft 24 distally, or may be driven proximally to move the elongate shaft 24 proximally. The handle 30 may be configured to be rotated about the longitudinal axis of the handle 30 and the elongate shaft 24 to rotate and torque the elongate shaft 24. Such rotation may be desired to provide for a desired orientation of the implant to be deployed from the implant retention area 32. The proximal portion 38 may include a flush port 42 for flushing fluid (including air) from the elongate shaft 24.

The handle 30 may further include a release mechanism positioned therein, which may be configured to release the implant from the implant retention area 32. Components of the release mechanism may include a release actuator 44 for being operated to release the implant from the implant retention area 32. The release actuator 44 may be positioned on the proximal portion 38 of the handle 30. The release mechanism may further include a lock on the proximal portion 38 for locking the release actuator 44 in position. The release mechanism may further include a motor or other driving device (such as a manually driven device) that may be positioned in the handle 30. The motor or other driving device may be configured to rotate a torque shaft to move an outer sheath that may cover the implant retention area 32.

The release mechanism may include a control device for operating the motor or other driving device. The control device may be in the form of buttons 52, 54 or other form of control device. One button 52 may be configured to retract the outer sheath 50 (move the outer sheath 50 proximally) and another button 54 may be configured to advance the outer sheath 50 (move the outer sheath 50 distally). As such, the control device may be used to selectively control deployment of the implant from the implant retention area 32 by movement of the outer sheath 50 (e.g., either exposing the implant for release or covering the implant for recapture). Other forms of release may include inflation of a balloon (for a balloon deployed implant) or operation of a mechanical drive to mechanically release the implant.

The handle 30 may include a deflection mechanism 58 configured to cause at least a portion of the elongate shaft 24 to deflect. The deflection may be in longitudinal planes extending outward from the longitudinal axis of the elongate shaft 24. Such deflection may be utilized to accommodate various bends in the patient's anatomy that may need to be navigated to deliver the implant to the desired location. The deflection mechanism 58 may provide for a controllable deflection of the elongate shaft 24, as opposed to a passive deflection that may occur by simply passing a flexible shaft through bends in the patient's anatomy.

The elongate shaft 24 may include multiple layers or multiple sheaths extending over other layers or sheaths. In some embodiments, an innermost component of the elongate shaft 24 may comprise an inner shaft or guidewire lumen 60. Such an inner shaft or guidewire lumen may further comprise a nose cone shaft of the elongate shaft that couples to the nose cone. The guidewire lumen 60 may extend for the length of the elongate shaft 24 and may have a proximal end that couples to the handle 30. A distal end of the guidewire lumen 60 may couple to the nose cone 34. A manifold may be coupled to the guidewire lumen 60 and retain sutures for retaining the implant in position within the implant retention area 32. A retainer may be coupled to the guidewire lumen 60 for coupling to release pins. Upon movement of the release pins, the implant may be allowed to deploy from the implant retention area 32.

A torque shaft may be provided comprising a sheath that extends over the guidewire lumen 60. The torque shaft may have a proximal end that couples to the motor or other driving device of the handle 30, and may have a distal portion that comprises a threaded portion for a threaded body (such as a nut or other form of threaded body) to slide along.

A first hypotube may be positioned distal of the distal end of the torque shaft and may be spaced from the distal end of the torque shaft. The first hypotube may include a plurality of cuts and may comprise a sheath extending over the guidewire lumen 60.

A pull tether coupler may be positioned within the first hypotube and may comprise a body configured to couple to a distal end of a pull tether and retain the pull tether to the first hypotube. The pull tether may have a distal end coupled to the pull tether coupler and a proximal portion including a proximal end coupled to the deflection mechanism.

A flex shaft 85 may comprise a sheath extending over the torque shaft and may have a proximal end coupled to the deflection mechanism and may have a distal end coupled to a proximal end of a torque shaft channel. The flex shaft 85 may comprise the substantial length of the elongate shaft 24 and forms an outer surface of the elongate shaft 24. The flex shaft 85 may be configured to be flexible to accommodate the anatomy of the patient's body being entered.

A second hypotube may have a proximal portion including the torque shaft channel. The torque shaft channel may comprise an opening allowing the threaded body to transmit motion provided from the threaded portion of the torque shaft to the outer sheath 50. The threaded body may slide longitudinally along the threaded portion of the torque shaft because the torque shaft channel may prevent rotation of the threaded body. The threaded body may extend outward through the torque shaft channel to couple to a proximal portion of a third hypotube, and thus cause the third hypotube to slide longitudinally along with the threaded body. As such, the outer sheath 50 is coupled to the third hypotube and is slid as well.

The second hypotube may have a distal portion including distal end that couples to a distal end of the first hypotube. As such, the second hypotube may couple to the pull tether and the first hypotube at the distal end of the second hypotube. A proximal portion of the second hypotube adjacent the torque shaft channel does not cover the first hypotube. The proximal portion of the second hypotube may include the proximal end of the first hypotube.

The outer sheath of the elongate shaft 24 may include multiple components. Such components may include the outer sheath 50 extending over the implant retention area 32. A proximal end of the outer sheath 50 may couple to a third hypotube, which may include a sheath 100 extending over the third hypotube and forming an outer surface of the elongate shaft 24. The proximal end of the third hypotube may couple to the threaded body, and such connection may be covered with an outer sheath 102 forming an outer surface of the elongate shaft 24. Further, a proximal portion of the outer sheath may include a fourth hypotube, which may impede fluid (such as blood) from entering into the torque shaft channel when the outer sheath is slid distally. The fourth hypotube may be covered with outer sheaths 106, 108 that form an outer surface of the elongate shaft 24.

In operation, the release mechanism may operate to rotate the torque shaft, which causes the implant to be exposed and deployed from the elongate shaft 24.

A deflection mechanism may be provided that operates to actively deflect at least a portion of the elongate shaft 24. The deflection mechanism may be positioned at a proximal end of the elongate shaft 24. The deflection mechanism may be positioned distal the handle 30 and at the proximate end of the elongate shaft 24, although other positions may be utilized as desired.

The deflection mechanism may include a control device that may be operated by an individual to control the deflection of the elongate shaft.

Conventionally, a retention ring may be fixedly attached around a distal end of the elongate shaft, for example by gluing or welding. The nose cone may then be overmolded upon the distal end of the elongate shaft, such that an internal retention channel of the nose cone is formed over the retention ring. Thus, the shape of the retention ring of the elongate shaft and the shape of the internal retention ring correspond to each other. The tight fit between the distal and proximal ends of the retention channel and the distal and proximal ends of the retention ring, respectively, prevent axial displacement of the nose cone relative to the elongate shaft. However, as noted herein, this process of overmolding the nose cone to the distal portion of the elongate shaft may be difficult to be performed after other portions of the delivery system are assembled, limiting the flexibility of assembly of the delivery system.

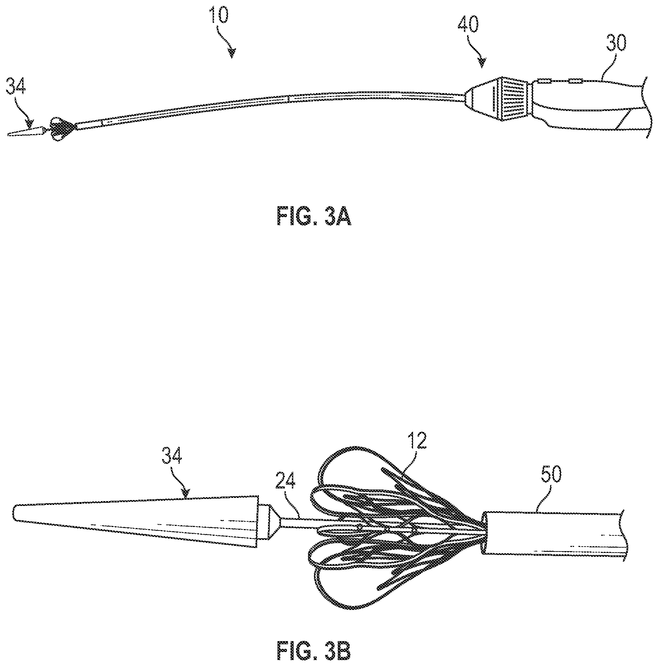

FIG. 3A shows a portion of delivery system 10. FIG. 3B shows a detailed view of the distal portion of the delivery system 10, including the outer sheath 50 being retracted to expose and deploy the implant 12.

As described herein, the conventional attachment of the nose cone 34 being overmolded to the elongate shaft 24 may dictate a specific delivery system assembly sequence. For example, in conventional systems, when components are added to the delivery system 10, the components are added at the proximal end of delivery system and may be slid in a distal direction along, for example, the elongate shaft 24. These components could not be attached at the distal end of the elongate shaft 24 and slid proximally because the nose cone is already attached and may block the components from being added in that direction. However, systems and methods described herein may allow components to be positioned at the distal end of the elongate shaft and slid proximally before the nose cone 34 is attached, along with other features and benefits.

Further, with the systems and methods described herein, the crimping of the implant to the elongate shaft may be performed prior to the attachment of the nose cone to the elongate shaft. This may prevent the nose cone from interfering with the crimping process and may allow the implant to be crimped effectively and accurately. The implant 12 may be crimped onto the elongate shaft 24 to reduce the widthwise profile of the implant 12. This crimping may be performed using a device that exerts pressure onto the implant 12 radially inward, toward the elongate shaft 24 in an even manner across the circumference of the implant 12. When a nose cone is already attached to the elongate shaft 24, as may be the case in conventional systems, the nose cone may interfere with the ability of the crimping device to crimp the implant, as the nose cone may become captured within the crimping device, limiting the range of motion of the crimping device onto the implant.

Further, when an implant is crimped, the crimping may cause the length of the implant to expand, as the width of the implant decreases. This increasing of length by the implant may be interfered with by a nose cone that is already attached to the elongate shaft. However, with the systems and methods described herein, the crimping of the implant to the elongate shaft may be performed prior to the attachment of the nose cone to the elongate shaft, which may prevent the nose cone from interfering with the crimping process.

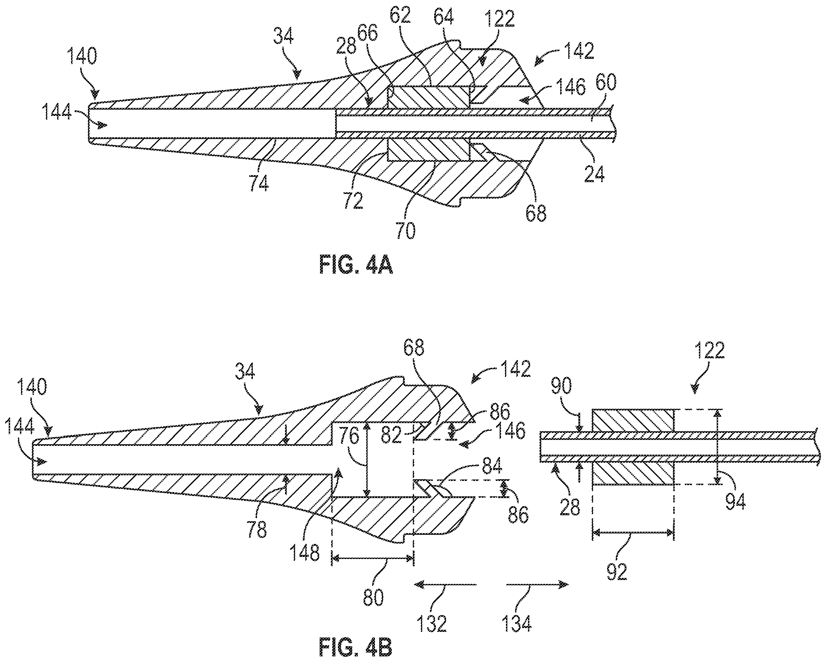

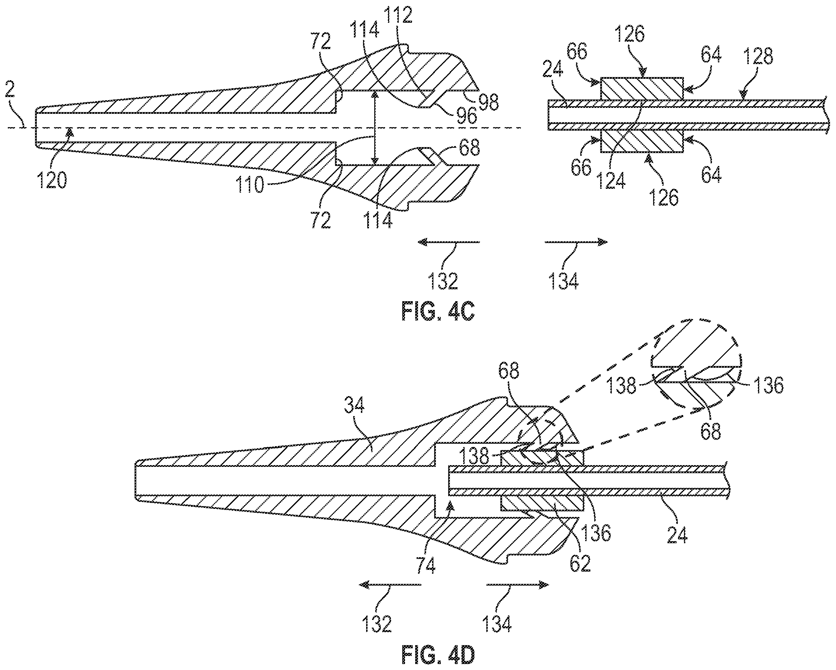

FIGS. 4A-4D illustrate side cross-sectional views along a centerline of a nose cone 34 and an elongate shaft 24. FIG. 4A illustrates the nose cone 34 coupled to the elongate shaft 24. FIGS. 4B and 4C illustrate the nose cone 34 and the elongate shaft 24 prior to coupling. FIG. 4D illustrates the nose cone 34 and the elongate shaft 24 during coupling. The system may include one or more angled projections 68 configured to couple the nose cone 34 to the distal end of the elongate shaft.

As shown in FIG. 4A, the elongate shaft 24 (which may have a guidewire lumen 60) may have a retention ring 62 located on a distal portion 122 of the elongate shaft 24. In particular, as shown in FIG. 4C, the retention ring 62 surrounds a portion of an outer surface 128 of the elongate shaft 24 such that an inner surface 124 of the retention ring 62 contacts the portion of the outer surface 128 of the elongate shaft 24. Referring back to FIG. 4A, the retention ring 62 is fixedly connected to the elongate shaft 24 such that the retention ring 62 is not capable of axial movement relative to the elongate shaft 24. The retention ring 62 may be connected to the elongate shaft 24 using an adhesive, welding, brazing, or any attachment technique. The retention ring 62 may have a distal end 66 and a proximal end 64.

The nose cone 34 may include a distal opening 144 at a distal end 140 of the nose cone 34 and a proximal opening 146 at a proximal end 142 of the nose cone 34. An inner surface 70 of the nose cone 34 defines a nose cone lumen 74 located between the distal opening 144 and the proximal opening 146. The distal opening 144 may be smaller in width than the proximal opening 146. The proximal opening 146 may be configured to receive the distal end 28 of the elongate shaft 24, along with a retention ring 62, into the nose cone lumen 74.

The nose cone 34 also includes one or more angled projections 68 located within the nose cone lumen 74 and extending radially inward from the inner surface 70 of the nose cone 34. The projections 68 may be formed integrally with the inner surface 70 of the nose cone 34. The projections 68 engage the retention ring 62 to prevent the retention ring 62 and the elongate shaft 24 from being removed from the proximal opening 146 of the nose cone 34. The projections 68 may be a single annular projection extending around an interior perimeter of the inner surface 70. Alternatively, the projections 68 may be multiple discrete projections spaced around the interior perimeter of the inner surface 70. These multiple discrete projections may be spaced evenly apart from each other to evenly distribute any load from the retention ring 62. Other configurations may be utilized as desired.

As shown in FIGS. 4B and 4C, the projections 68 may be angled toward the distal end 140 of the nose cone 34. The projections 68 have a proximal side 96 facing the proximal end 142 of the nose cone 34 and a distal side 112 facing the distal end 140 of the nose cone 34. A distal projection angle 82 is defined by the distal side 112 of the projection 68 and the inner surface 70 of the nose cone 34. A proximal projection angle 84 is defined by the proximal side 96 and the inner surface 70 of the nose cone 34. The inner surface 70 of the nose cone 34 may be further divided into a distal portion 110 located between the projections 68 and a wall 72 and a proximal portion 98 located between the projections 68 and the proximal opening 146. Thus, the distal projection angle 82 may be defined by the distal side 112 of the projection 68 and the distal portion 110 of the inner surface 70 and the proximal projection angle 84 may be defined by the proximal side 96 and the proximal portion 98 of the inner surface 70. The distal projection angle 82 may be an acute angle and the proximal projection angle 84 may be an obtuse angle.

The projections 68 may have a neutral position when no force is exerted upon the projections 68. From the neutral position, the projections 68 are capable of bending or pivoting in the distal direction 132 to allow the retention ring 62 to pass into the nose cone lumen 74. This bending or pivoting of the projections 68 in the distal direction 132 causes the distal projection angle 82 to be reduced relative to the neutral position and the proximal projection angle 84 to be increased relative to the neutral position. The projections 68 may be biased (e.g., spring-biased) to return to its neutral position after being bent or pivoted in the distal direction 132. The biasing may be due to the rigidity of the material the nose cone 34 is made of. This may allow the projections 68 to secure the retention ring 62 after the retention ring 62 is fully received in a retention ring portion 148 of the nose cone lumen 74. In contrast, from the neutral position, the projections 68 may be incapable of or resistant to bending or pivoting in the proximal direction 134. This inability or resistance allows the projections 68 to retain, secure, and hold the retention ring 62 within the nose cone lumen 74.

The projections 68 have a height 86, which is the distance between the inner surface 70 and the tip 114 of the projections 68. The height 86 of the projections 68 may be optimized to provide retention force onto the retention ring 62 when the retention ring 62 exerts force on the projections 68 in the proximal direction 134 and also optimized to allow ease of clearance of the projections 68 by the retention ring 62 when the retention ring 62 is being mated with the nose cone 34. Due to the angled orientation of the projections 68, the height 86 is less than the actual length of the projections 68.

The nose cone 34 may have a shaft tip portion 120 and a retention ring portion 148. The shaft tip portion 120 is configured to receive the distal end 28 of the elongate shaft 24 when the elongate shaft 24 and the nose cone 34 are mated (as shown in FIG. 4A) and the retention ring portion 148 is configured to receive the retention ring 62 when the elongate shaft 24 and the nose cone 34 are mated (as shown in FIG. 4A). The shaft tip portion 120 has a width 78 and the retention ring portion 148 has a width 76. The width 76 of the retention ring portion 148 is larger than the width 78 of the shaft tip portion 120.

The retention ring portion 148 also may have a length 80 that is defined by a wall 72 and a tip 114 of the projections 68. The wall 72 may connect the inner surface 70 of the retention ring portion 148 to the inner surface 70 of the shaft tip portion 120. The wall 72 may contact the distal end 66 of the retention ring 62 when the elongate shaft 24 and the nose cone 34 are mated. The tip 114 contacts the proximal end 64 of the retention ring 62 when the elongate shaft 24 and the nose cone 34 are mated. The wall 72 may be located on a plane perpendicular to a lengthwise axis 2 that the nose cone 34 lies upon. In some embodiments, the wall 72 is angled relative to the lengthwise axis 2 that the nose cone 34 lies upon, facing the proximal opening 146. In these embodiments, the distal end 66 of the retention ring 62 may also be angled in a similar manner (angled relative to the lengthwise axis 2 that the nose cone 34 lies upon). The angles of the distal end 66 of the retention ring 62 and the wall 72 may serve as a guide to align the retention ring 62 within the nose cone lumen 74, in situations where the width 94 of the retention ring 62 is less than the width 76 of the retention ring portion 148 and the retention ring 62 may move radially within the retention ring portion 148.

The elongate shaft 24 has a width 90 that is less than the width 78 of the shaft tip portion 120, allowing the shaft tip portion 120 to receive the elongate shaft 24. The retention ring 62 has a width 94 that is less than the width 76 of the retention ring portion 148 and a length 92 that is less than the length 80 of the retention ring portion 148, allowing the retention ring portion 148 to receive and house the retention ring 62.

The elongate shaft 24 and the retention ring 62 are moved in a distal direction 132 (opposite a proximal direction 134) approaching the proximal end 142 of the nose cone 34, to couple the elongate shaft 24 to the nose cone 34. As such, a translation of the proximal end of the nose cone relative to the distal end of the elongate shaft couples the nose cone to the distal end of the elongate shaft.

As shown in FIG. 4D, the nose cone 34 has partially received the elongate shaft 24 and the retention ring 62. The projections 68 are bent or pivoted in the distal direction 132 to allow the retention ring 62 to pass into the nose cone lumen 74. This bending or pivoting of the projections 68 in the distal direction 132 causes a distal projection angle 138 that is less than the distal projection angle 82 of the neutral position and also causes a proximal projection angle 136 that is greater than the proximal projection angle 84 of the neutral position.

With reference to FIGS. 4A and 4C, when the retention ring 62 and the elongate shaft 24 are mated with the nose cone 34, the wall 72 may contact the distal end 66 of the retention ring 62, the tip 114 may contact the proximal end 64 of the retention ring 62, and the inner surface 70 may contact the outer surface 126 of the retention ring 62. The contacting of the wall 72 by the distal end 66 of the retention ring 62 prevents further axial movement of the elongate shaft 24 in the distal direction 132. The contacting of the tip 114 by the proximal end 64 of the retention ring 62 prevents further axial movement of the elongate shaft 24 in the proximal direction 134. The contacting of the inner surface 70 in the retention ring portion 148 (in particular, the distal portion 110) by the outer surface 126 of the retention ring 62 prevents radial movement of the elongate shaft 24.

In some embodiments, a guide wire may pass through the guidewire lumen 60 of the elongate shaft 24, out of the distal end 28 of the elongate shaft, through the nose cone lumen 74, and out of the distal opening 144 of the nose cone 34. The delivery system 10 may be guided by the guide wire to a location within the patient for installation of the implant 12. In other embodiments, a guide wire is not used, and the elongate shaft 24 may be solid, and not hollow.

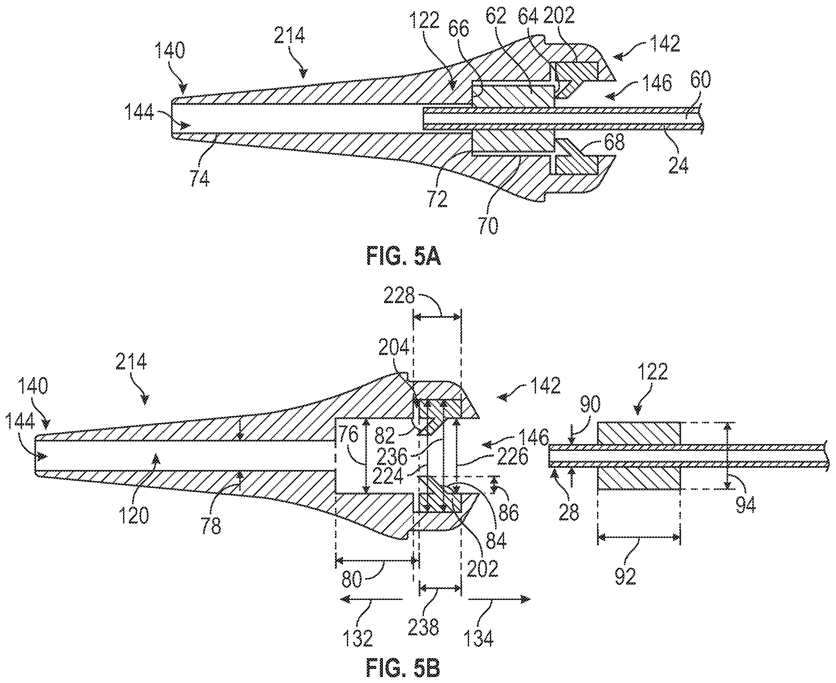

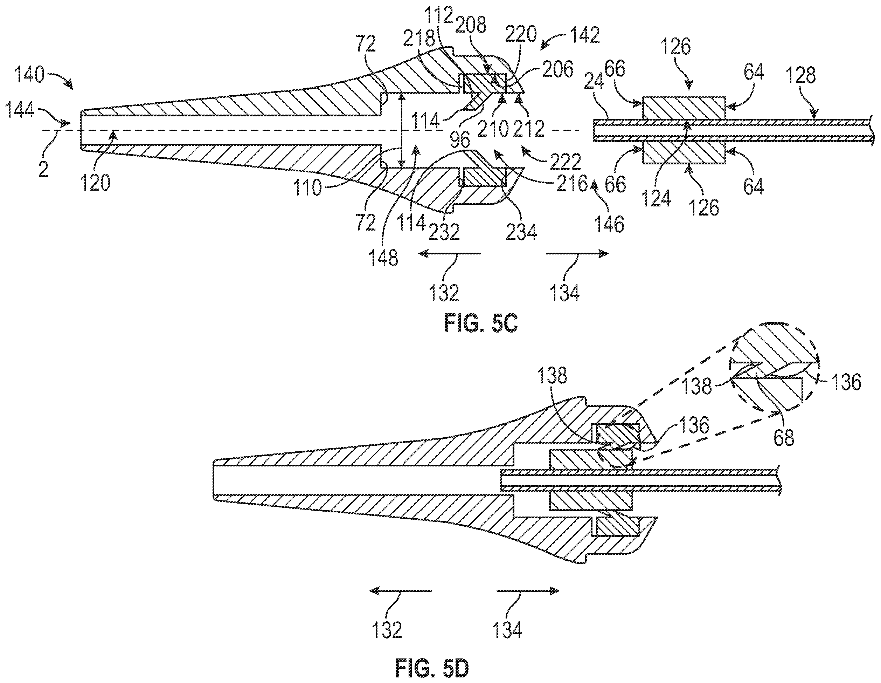

FIGS. 5A-5D illustrate side cross-sectional views along a centerline of a nose cone 214 and an elongate shaft 24. FIG. 5A illustrates the nose cone 214 being coupled to the elongate shaft 24. FIGS. 5B and 5C illustrate the nose cone 214 and the elongate shaft 24 prior to coupling. FIG. 5D illustrates the nose cone 214 and the elongate shaft 24 during coupling. Nose cone 214 is similar to nose cone 34 with different portions numbered differently and similar portions numbered similarly. The system may include one or more angled projections 68 configured to couple the nose cone 214 to the distal end of the elongate shaft.

As shown in FIG. 5A, the elongate shaft 24 has a retention ring 62 located on a distal portion 122 of the elongate shaft 24. In particular, as shown in FIG. 5C, the retention ring 62 surrounds a portion of an outer surface 128 of the elongate shaft 24 such that an inner surface 124 of the retention ring 62 contacts the portion of the outer surface 128 of the elongate shaft 24. Referring back to FIG. 5A, the retention ring 62 is fixedly connected to the elongate shaft 24 such that the retention ring 62 is not capable of axial movement relative to the elongate shaft 24. The retention ring 62 may be connected to the elongate shaft 24 using an adhesive, welding, brazing, or any attachment technique. The retention ring 62 has a distal end 66 and a proximal end 64.

The nose cone 214 includes a distal opening 144 at a distal end 140 of the nose cone 214 and a proximal opening 146 at a proximal end 142 of the nose cone 214. An inner surface 70 of the nose cone 214 defines a nose cone lumen 74 located between the distal opening 144 and the proximal opening 146. The distal opening 144 is smaller in width than the proximal opening 146. The proximal opening 146 is configured to receive the distal end 28 of the elongate shaft 24, along with a retention ring 62, into the nose cone lumen 74.

Located within the nose cone lumen 74 is a support ring 202. The support ring 202 includes projections 68 located within the nose cone lumen 74 and extending radially inward from the inner surface 210 of the support ring 202. The projections 68 engage the retention ring 62 to prevent the retention ring 62 and the elongate shaft 24 from being removed from the proximal opening 146 of the nose cone 214. The projections 68 may be a single annular projection extending around an interior perimeter of the inner surface 70. Alternatively, the projections 68 may be multiple discrete projections spaced around the interior perimeter of the inner surface 70. These multiple discrete projections may be spaced evenly apart from each other to evenly distribute any load from the retention ring 62. Other configurations of projections may be utilized as desired.

As shown in FIGS. 5B and 5C, the projections 68 are angled toward the distal end 140 of the nose cone 214. The projections 68 have a proximal side 96 facing the proximal end 142 of the nose cone 214 and a distal side 112 facing the distal end 140 of the nose cone 214. A distal projection angle 82 is defined by the distal side 112 of the projection 68 and the inner surface 210 of the support ring 202. A proximal projection angle 84 is defined by the proximal side 96 and the inner surface 210 of the support ring 202. The distal projection angle 82 may be an acute angle and the proximal projection angle 84 may be an obtuse angle.

The projections 68 may have a neutral position when no force is exerted upon the projections 68. From the neutral position, the projections 68 are capable of bending or pivoting in the distal direction 132 to allow the retention ring 62 to pass into the nose cone lumen 74. This bending or pivoting of the projections 68 in the distal direction 132 causes the distal projection angle 82 to be reduced relative to the neutral position and the proximal projection angle 84 to be increased relative to the neutral position. The projections 68 may be biased (e.g., spring-biased) to return to its neutral position after being bent or pivoted in the distal direction 132. The biasing may be due to the rigidity of the material the support ring 202 is made of. This allows the projections 68 to secure the retention ring 62 after the retention ring 62 is fully received in the retention ring portion 148 of the nose cone lumen 74. In contrast, from the neutral position, the projections 68 may be incapable of or resistant to bending or pivoting in the proximal direction 134. This inability or resistance allows the projections 68 to retain, secure, and hold the retention ring 62 within the nose cone lumen 74.

The projections 68 have a height 86, which is the distance between the inner surface 210 and the tip 114 of the projections 68. The height 86 of the projections 68 may be optimized to provide retention force onto the retention ring 62 when the retention ring 62 exerts force on the projections 68 in the proximal direction 134 and also optimized to allow ease of clearance of the projections 68 by the retention ring 62 when the retention ring 62 is being mated with the nose cone 214. Due to the angled orientation of the projections 68, the height 86 is less than the actual length of the projections 68.

The nose cone 214 may have a shaft tip portion 120, a retention ring portion 148, a support ring portion 216, and a lip portion 222. The shaft tip portion 120 is configured to receive the distal end 28 of the elongate shaft 24 when the elongate shaft 24 and the nose cone 214 are mated (as shown in FIG. 5A). The retention ring portion 148 is configured to receive the retention ring 62 when the elongate shaft 24 and the nose cone 214 are mated (as shown in FIG. 5A). The support ring portion 216 is configured to receive the support ring 202. The lip portion 222 is at the proximal end 142 of the nose cone 214 and is configured to secure the support ring 202 and prevent the support ring 202 from being removed from the proximal opening 146 of the nose cone 214.

The shaft tip portion 120 has a width 78, the retention ring portion 148 has a width 76, the support ring portion 216 has a width 224, and the lip portion 222 has a width 226. The width 76 of the retention ring portion 148 is larger than the width 78 of the shaft tip portion 120. The width 224 of the support ring portion 216 is larger than the width 76 of the retention ring portion 148. The width 226 of the lip portion 222 is the same as the width 76 of the retention ring portion 148.

The retention ring portion 148 has a length 80 that is defined by a wall 72 and a tip 114 of the projections 68. The wall 72 connects an inner surface 70 of the retention ring portion 148 to the inner surface 70 of the shaft tip portion 120. The wall 72 contacts the distal end 66 of the retention ring 62 when the elongate shaft 24 and the nose cone 214 are mated. The tip 114 contacts the proximal end 64 of the retention ring 62 when the elongate shaft 24 and the nose cone 214 are mated. The wall 72 may be located on a plane perpendicular to a lengthwise axis 2 that the nose cone 214 lies upon. In some embodiments, the wall 72 is angled relative to the lengthwise axis 2 that the nose cone 214 lies upon, facing the proximal opening 146. In these embodiments, the distal end 66 of the retention ring 62 may also be angled in a similar manner (angled relative to the lengthwise axis 2 that the nose cone 214 lies upon). The angles of the distal end 66 of the retention ring 62 and the wall 72 may serve as a guide to align the retention ring 62 within the nose cone lumen 74, in situations where the width 94 of the retention ring 62 is less than the width 76 of the retention ring portion 148 and the retention ring 62 may move radially within the retention ring portion 148.

The support ring portion 216 has a length 228 that is defined by a wall 232 and a wall 234. The wall 232 connects an inner surface 70 of the retention ring portion 148 to the inner surface 70 of the support ring portion 216 (also referred to as the inner surface 206 of the support ring portion 216). The wall 234 connects an inner surface 70 of the support ring portion 216 and the inner surface 70 of the lip portion 222 (also referred to as the inner surface 212 of the lip portion 222). The proximal end 220 of the support ring 202 contacts the wall 234. In some embodiments, gap 204 is located between the distal end 218 of the support ring 202 and the wall 232. In other embodiments, there is no gap 204, and the distal end 218 of the support ring 202 contacts the wall 232.

The support ring 202 has an outer surface 208 that contacts the inner surface 206 of the support ring portion 216. The support ring 202 may be held within the support ring portion 216 by an adhesive, interference fit, or any other technique. In some embodiments, the support ring 202 is placed within the support ring portion 216 without being attached to the nose cone 214 via an adhesive. The support ring 202 may be capable of deforming, such that it is compressed to fit within the proximal opening 146 and placed within the support ring portion 216, where it expands to occupy the space within the support ring portion 216. In some embodiments, the support ring 202 is fabricated first, and the nose cone 214 is then fabricated around the support ring 202.

The elongate shaft 24 has a width 90 that is less than the width 78 of the shaft tip portion 120, allowing the shaft tip portion 120 to receive the elongate shaft 24. The retention ring 62 has a width 94 that is less than the width 76 of the retention ring portion 148 and a length 92 that is less than the length 80 of the retention ring portion 148, allowing the retention ring portion 148 to receive and house the retention ring 62. The support ring 202 has a width 236 that is the same as the width 224 of the support ring portion 216. The support ring 202 has a length 238 that is less than the length 228 of the support ring portion 216. In some embodiments, the length 238 of the support ring 202 is the same as the length 228 of the support ring portion 216.

The elongate shaft 24 and the retention ring 62 are moved in a distal direction 132 (opposite a proximal direction 134) toward the proximal end 142 of the nose cone 214, to connect the elongate shaft 24 to the nose cone 214. As such, a translation of the proximal end of the nose cone relative to the distal end of the elongate shaft couples the nose cone to the distal end of the elongate shaft.

As shown in FIG. 5D, the nose cone 214 has partially received the elongate shaft 24 and the retention ring 62. The projections 68 are bent or pivoted in the distal direction 132 to allow the retention ring 62 to pass into the nose cone lumen 74. This bending or pivoting of the projections 68 in the distal direction 132 causes a distal projection angle 138 that is less than the distal projection angle 82 of the neutral position and also causes a proximal projection angle 136 that is greater than the proximal projection angle 84 of the neutral position.

With reference to FIGS. 5A and 5C, when the retention ring 62 and the elongate shaft 24 are mated with the nose cone 214, the wall 72 may contact the distal end 66 of the retention ring 62, the tip 114 may contact the proximal end 64 of the retention ring 62, and the inner surface 70 may contact the outer surface 126 of the retention ring 62. The contacting of the wall 72 by the distal end 66 of the retention ring 62 may prevent further axial movement of the elongate shaft 24 in the distal direction 132. The contacting of the tip 114 by the proximal end 64 of the retention ring 62 may prevent further axial movement of the elongate shaft 24 in the proximal direction 134. The contacting of the inner surface 70 in the retention ring portion 148 (in particular, the distal portion 110) by the outer surface 126 of the retention ring 62 may prevent radial movement of the elongate shaft 24.

In some embodiments, a guide wire may pass through the guidewire lumen 60 of the elongate shaft 24, out of the distal end 28 of the elongate shaft, through the nose cone lumen 74, and out of the distal opening 144 of the nose cone 214. The delivery system 10 may be guided by the guide wire to a location within the patient for installation of the implant 12. In other embodiments, a guide wire is not used, and the elongate shaft 24 may be solid, and not hollow.

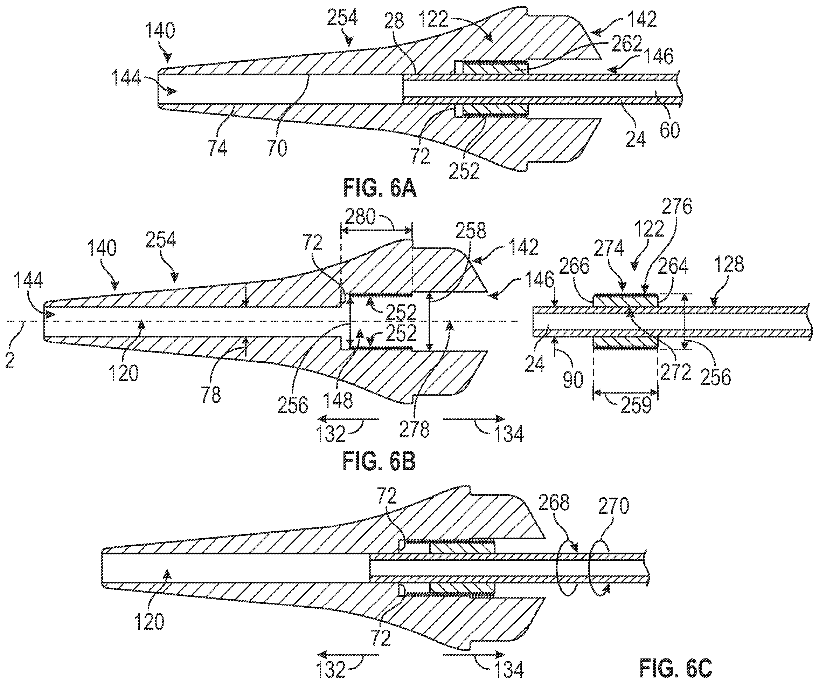

FIGS. 6A-6C illustrate side cross-sectional views along a centerline of a nose cone 254 and an elongate shaft 24. FIG. 6A illustrates the nose cone 254 coupled to the elongate shaft 24. FIG. 6B illustrates the nose cone 254 and the elongate shaft 24 prior to coupling. FIG. 6C illustrates the nose cone 254 and the elongate shaft 24 during coupling. The system may include a threaded coupler configured to couple the nose cone to the distal end of the elongate shaft.

As shown in FIG. 6A, the elongate shaft 24 has a retention ring 262 located on a distal portion 122 of the elongate shaft 24. In particular, as shown in FIG. 6B, the retention ring 262 surrounds a portion of an outer surface 128 of the elongate shaft 24 such that an inner surface 272 of the retention ring 262 contacts the portion of the outer surface 128 of the elongate shaft 24. Referring back to FIG. 6A, the retention ring 262 is fixedly connected to the elongate shaft 24 such that the retention ring 262 is not capable of axial movement relative to the elongate shaft 24. The retention ring 262 may be connected to the elongate shaft 24 using an adhesive, welding, brazing, or any attachment technique. The retention ring 262 has a distal end 266 and a proximal end 264.

The nose cone 254 includes a distal opening 144 at a distal end 140 of the nose cone 254 and a proximal opening 146 at a proximal end 142 of the nose cone 254. An inner surface 70 of the nose cone 254 defines a nose cone lumen 74 located between the distal opening 144 and the proximal opening 146. The distal opening 144 is smaller in width than the proximal opening 146. The proximal opening 146 is configured to receive the distal end 28 of the elongate shaft 24, along with a retention ring 262, into the nose cone lumen 74.

As shown in FIG. 6B, the threaded coupler may include a set of nose cone threads 252 located within the nose cone lumen 74 and extending radially inward from the inner surface 70 of the nose cone 254. The nose cone threads 252 may be formed integrally with the inner surface 70 of the nose cone 254. The nose cone threads 252 are configured to engage a set of corresponding retention ring threads 274 of the threaded coupler to prevent the retention ring 262 and the elongate shaft 24 from being removed from the proximal opening 146 of the nose cone 254. The retention ring threads 274 are located on an outer surface 276 of the retention ring 262. The retention ring threads 274 may cover an entire length of the retention ring 262 or may only cover a portion of the length of the retention ring 262.

The nose cone 254 may have a shaft tip portion 120, a retention ring portion 148, and an opening portion 278. The shaft tip portion 120 is configured to receive the distal end 28 of the elongate shaft 24 when the elongate shaft 24 and the nose cone 254 are mated (as shown in FIG. 6A). The retention ring portion 148 is configured to receive the retention ring 262 when the elongate shaft 24 and the nose cone 254 are mated (as shown in FIG. 6A). The opening portion 278 connects the retention ring portion 148 to the proximal opening 146. The shaft tip portion 120 has a width 78, the retention ring portion 148 has a width 256, and the opening portion 278 has a width 258. The width 256 of the retention ring portion 148 is larger than the width 78 of the shaft tip portion 120. The width 258 of the opening portion 278 is larger than or equal to the width 256 of the retention ring portion 148.

The retention ring portion 148 also has a length 280 that is defined by a wall 72 and the end of the nose cone threads 252. The wall 72 connects the inner surface 70 of the retention ring portion 148 to the inner surface 70 of the shaft tip portion 120. The wall 72 may contact the distal end 266 of the retention ring 262 when the elongate shaft 24 and the nose cone 254 are mated. The opening portion 278 is located between the proximal end of the nose cone threads 252 and the proximal opening 146.

The elongate shaft 24 has a width 90 that is less than the width 78 of the shaft tip portion 120, allowing the shaft tip portion 120 to receive the elongate shaft 24. The retention ring 262 has a width 256 that corresponds to the width 256 of the retention ring portion 148 to ensure contact and security between the nose cone threads 252 and the retention ring threads 274. The retention ring 262 has a length 259 that corresponds to the length 280 of the retention ring portion 148, to promote contact and security between the nose cone threads 252 and the retention ring threads 274.

The elongate shaft 24 and the retention ring 262 are moved in a distal direction 132 (opposite a proximal direction 134) toward the proximal end 142 of the nose cone 254, to connect the elongate shaft 24 to the nose cone 254. As such, a translation of the proximal end of the nose cone relative to the distal end of the elongate shaft couples the nose cone to the distal end of the elongate shaft.

As shown in FIG. 6C, the nose cone 254 has partially received the elongate shaft 24 and the retention ring 262. When the nose cone threads 252 and the retention ring threads 274 make contact, the elongate shaft 24 (or the nose cone 254) may be rotated in a first direction 268 to advance the retention ring 262 in a distal direction 132 along the nose cone threads 252 and the retention ring threads 274. A portion of the nose cone threads 252 and a portion of the retention ring threads 274 are engaged. In particular a portion of the nose cone threads 252 that are on the proximal end of the nose cone threads 252 and a portion of the retention ring threads 274 that are on the distal end of the retention ring threads 274 are engaged. The elongate shaft 24 may be rotated in a second direction 270 to remove the retention ring 262 in a proximal direction 134 along the nose cone threads 252 and the retention ring threads 274. The interlocking surfaces of the nose cone threads 252 and the retention ring threads 274 allow the elongate shaft 24 to be secured to the nose cone 254. The first direction 268 and the second direction 270 are about a lengthwise axis 2 of the nose cone 254.

In some embodiments, a guide wire may pass through the guidewire lumen 60 of the elongate shaft 24, out of the distal end 28 of the elongate shaft, through the nose cone lumen 74, and out of the distal opening 144 of the nose cone 254. The delivery system 10 may be guided by the guide wire to a location within the patient for installation of the implant 12. In other embodiments, a guide wire is not used, and the elongate shaft 24 may be solid, and not hollow.

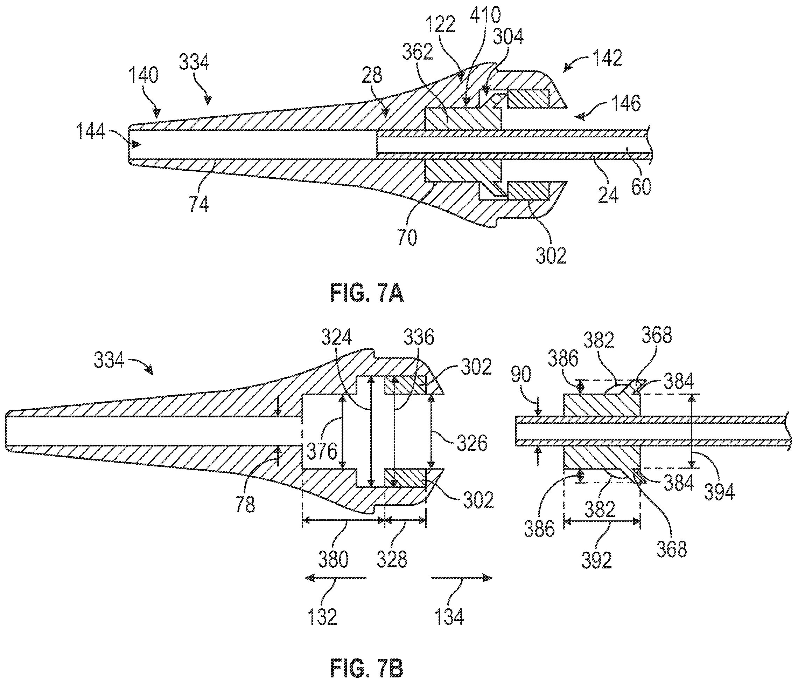

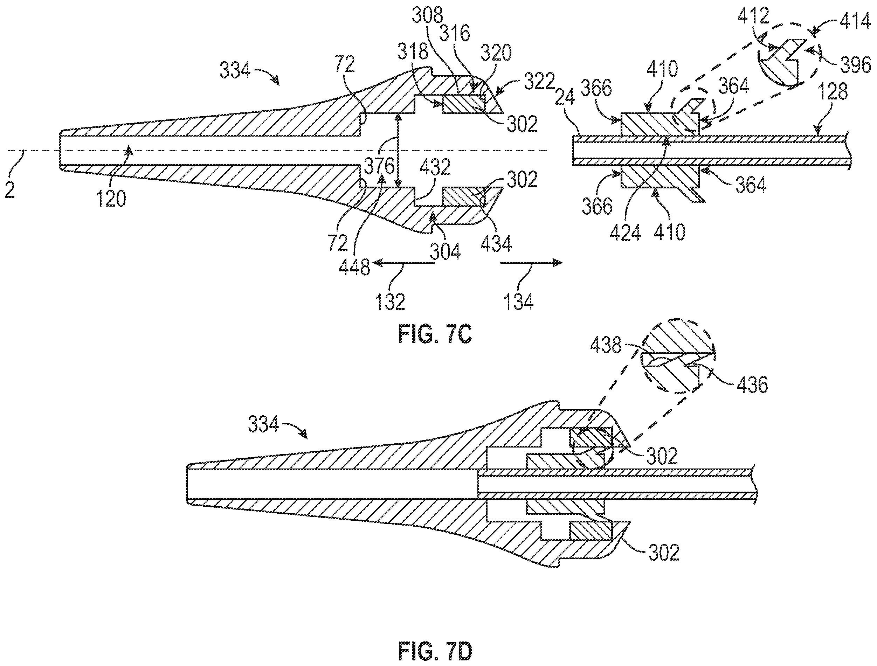

FIGS. 7A-7D illustrate side cross-sectional views along a centerline of a nose cone 334 and an elongate shaft 24. FIG. 7A illustrates the nose cone 334 coupled to the elongate shaft 24. FIGS. 7B and 7C illustrate the nose cone 334 and the elongate shaft 24 prior to coupling. FIG. 7D illustrates the nose cone 334 and the elongate shaft 24 during coupling. Nose cone 334 is similar to nose cone 34 with different portions numbered differently and similar portions numbered similarly. The system may include one or more angled projections 368 configured to couple the nose cone 334 to the distal end of the elongate shaft.

As shown in FIG. 7A, the elongate shaft 24 has a retention ring 362 located on a distal portion 122 of the elongate shaft 24. In particular, as shown in FIG. 7C, the retention ring 362 surrounds a portion of an outer surface 128 of the elongate shaft 24 such that an inner surface 424 of the retention ring 362 contacts the portion of the outer surface 128 of the elongate shaft 24. Referring back to FIG. 7A, the retention ring 362 is fixedly connected to the elongate shaft 24 such that the retention ring 362 is not capable of axial movement relative to the elongate shaft 24. The retention ring 362 may be connected to the elongate shaft 24 using an adhesive, welding, brazing, or any attachment technique. As shown in FIG. 7C, the retention ring 362 has a distal end 366 and a proximal end 364.

Referring back to FIG. 7A, the nose cone 334 includes a distal opening 144 at a distal end 140 of the nose cone 334 and a proximal opening 146 at a proximal end 142 of the nose cone 334. An inner surface 70 of the nose cone 334 defines a nose cone lumen 74 located between the distal opening 144 and the proximal opening 146. The distal opening 144 is smaller in width than the proximal opening 146. The proximal opening 146 is configured to receive the distal end 28 of the elongate shaft 24, along with a retention ring 362, into the nose cone lumen 74.

Located within the nose cone lumen 74 is a support ring 302. The support ring 302 and a wall of the inner surface 70 of the nose cone 334 forms a gap 304. In some embodiments, there is no support ring 302 and the nose cone 334 is dimensioned to include the gap 304 as part of the features of the inner surface 70 of the nose cone 334, obviating the need for a separate support ring 302.

The retention ring 362 may include projections 368 extending radially outward from an outer surface 410 of the retention ring 362. The projections 368 engage the gap 304 to prevent the retention ring 362 and the elongate shaft 24 from being removed from the proximal opening 146 of the nose cone 334. The projections 368 may be a single annular projection extending around an outer perimeter of the outer surface 410 of the retention ring 362. Alternatively, the projections 368 may be multiple discrete projections spaced around the outer perimeter of the outer surface 410. These multiple discrete projections may be spaced evenly apart from each other to evenly distribute any load. Other configurations may be utilized as desired.

As shown in FIGS. 7B and 7C, the retention ring 362 has a distal end 366 and a proximal end 364. The projections 368 are angled toward the proximal end 364 of the retention ring 362. Accordingly, when the retention ring 362 is mated with the nose cone 334, the projections 368 are angled toward the proximal end 142 of the nose cone 334. The projections 368 have a proximal side 396 facing the proximal end 364 of the retention ring 362 and a distal side 412 facing the distal end 366 of the retention ring 362. A distal projection angle 382 is defined by the distal side 412 of the projection 368 and the outer surface 410 of the retention ring 362. A proximal projection angle 384 is defined by the proximal side 396 and the outer surface 410 of the retention ring 362. The distal projection angle 382 may be an obtuse angle and the proximal projection angle 384 may be an acute angle.

The projections 368 may have a neutral position when no force is exerted upon the projections 368. From the neutral position, the projections 368 are capable of bending or pivoting in the proximal direction 134 to allow the retention ring 362 to pass into the nose cone lumen 74. This bending or pivoting of the projections 368 in the proximal direction 134 causes the proximal projection angle 384 to be reduced relative to the neutral position and the distal projection angle 382 to be increased relative to the neutral position. The projections 368 may be biased (e.g., spring-biased) to return to its neutral position after being bent or pivoted in the proximal direction 134. The biasing may be due to the rigidity of the material the retention ring 362 is made of. This allows the projections 368 to secure the support ring 302 after the retention ring 362 is fully received in the retention ring portion 448 of the nose cone lumen 74. In contrast, from the neutral position, the projections 368 may be incapable of or resistant to bending or pivoting in the distal direction 132. This inability or resistance allows the projections 368 to retain, secure, and hold the retention ring 362 within the nose cone lumen 74.

The projections 368 have a height 386, which is the distance between the outer surface 410 and the tip 414 of the projections 368. The height 386 of the projections 368 may be optimized to provide retention force onto the support ring 302 when the retention ring 362 exerts force on the projections 368 in the proximal direction 134 and also optimized to allow ease of clearance of the projections 368 past the support ring 302 and the proximal opening 146 when the retention ring 362 is being mated with the nose cone 334. Due to the angled orientation of the projections 368, the height 386 is less than the actual length of the projections 368.

The nose cone 334 may have a shaft tip portion 120, a retention ring portion 148, a support ring portion 316, and a lip portion 322. The shaft tip portion 120 is configured to receive the distal end 28 of the elongate shaft 24 when the elongate shaft 24 and the nose cone 334 are mated (as shown in FIG. 5A). The retention ring portion 448 is configured to receive the retention ring 362 when the elongate shaft 24 and the nose cone 334 are mated (as shown in FIG. 5A). The support ring portion 316 is configured to receive the support ring 302. The lip portion 322 is at the proximal end 142 of the nose cone 334 and is configured to secure the support ring 302 and prevent the support ring 302 from being removed from the proximal opening 146 of the nose cone 334.

The shaft tip portion 120 has a width 78, the retention ring portion 448 has a width 376, the support ring portion 316 has a width 324, and the lip portion 322 has a width 326. The width 376 of the retention ring portion 448 is larger than the width 78 of the shaft tip portion 120. The width 324 of the support ring portion 316 is larger than the width 376 of the retention ring portion 448. The width 326 of the lip portion 322 is the same as the width 376 of the retention ring portion 448.

The retention ring portion 448 has a length 380 that is defined by a wall 72 and a distal end 318 of the support ring 302. The wall 72 connects an inner surface 70 of the retention ring portion 448 to the inner surface 70 of the shaft tip portion 120. The wall 72 contacts the distal end 366 of the retention ring 362 when the elongate shaft 24 and the nose cone 334 are mated. The wall 72 may be located on a plane perpendicular to a lengthwise axis 2 that the nose cone 334 lies upon. In some embodiments, the wall 72 is angled relative to the lengthwise axis 2 that the nose cone 334 lies upon, facing the proximal opening 146. In these embodiments, the distal end 366 of the retention ring 362 may also be angled in a similar manner (angled relative to the lengthwise axis 2 that the nose cone 334 lies upon). The angles of the distal end 366 of the retention ring 362 and the wall 72 may serve as a guide to align the retention ring 362 within the nose cone lumen 74, in situations where the width 394 of the retention ring 362 is less than the width 376 of the retention ring portion 448 and the retention ring 362 may move radially within the retention ring portion 448.

The support ring portion 316 has a length 328 that is defined by the distal end 318 of the support ring 302 and a wall 434. The wall 434 connects an inner surface 70 of the support ring portion 316 and the inner surface 70 of the lip portion 322. The proximal end 320 of the support ring 302 contacts the wall 434.

The gap 304 is located between a wall 432 and the distal end 318 of the support ring 302. The wall 432 connects an inner surface 70 of the retention ring portion 448 to the inner surface 70 of the support ring portion 316.

The support ring 302 has an outer surface 308 that contacts the inner surface 70 of the support ring portion 316. The support ring 302 may be held within the support ring portion 316 by an adhesive, interference fit, or any other technique. In some embodiments, the support ring 302 is placed within the support ring portion 316 without being attached to the nose cone 334 via an adhesive. The support ring 302 may be capable of deforming, such that it is compressed to fit within the proximal opening 146 and placed within the support ring portion 316, where it expands to occupy the space within the support ring portion 316. In some embodiments, the support ring 302 is fabricated first, and the nose cone 334 is fabricated around the support ring 302.

The elongate shaft 24 has a width 90 that is less than the width 78 of the shaft tip portion 120, allowing the shaft tip portion 120 to receive the elongate shaft 24. The retention ring 362 has a width 394 that is less than the width 376 of the retention ring portion 448 and a length 392, allowing the retention ring portion 448 to receive and house the retention ring 362. The support ring 302 has a width 336 that is the same as the width 324 of the support ring portion 316. The support ring 302 has a length that corresponds to the length 328 of the support ring portion 316.

The elongate shaft 24 and the retention ring 62 are moved in a distal direction 132 (opposite a proximal direction 134) toward the proximal end 142 of the nose cone 334, to connect the elongate shaft 24 to the nose cone 334. As such, a translation of the proximal end of the nose cone relative to the distal end of the elongate shaft couples the nose cone to the distal end of the elongate shaft.

As shown in FIG. 7D, the nose cone 334 has partially received the elongate shaft 24 and the retention ring 362. The projections 368 are bent or pivoted in the proximal direction 134 to allow the retention ring 362 to pass into the nose cone lumen 74. This bending or pivoting of the projections 368 in the proximal direction 134 causes a distal projection angle 438 that is greater than the distal projection angle 382 of the neutral position and also causes a proximal projection angle 436 that is greater than the proximal projection angle 384 of the neutral position.

With reference to FIGS. 7A and 7C, when the retention ring 362 and the elongate shaft 24 are mated with the nose cone 334, the wall 72 may contact the distal end 366 of the retention ring 362, the tip 414 may contact the distal end 318 of the support ring 302, and the inner surface 70 may contact the outer surface 410 of the retention ring 362. The contacting of the wall 72 by the distal end 366 of the retention ring 362 may prevent further axial movement of the elongate shaft 24 in the distal direction 132. The contacting of the tip 114 by the distal end 318 of the support ring 302 prevents further axial movement of the elongate shaft 24 in the proximal direction 134. The contacting of the inner surface 70 in the retention ring portion 148 by the outer surface 410 of the retention ring 362 prevents radial movement of the elongate shaft 24.

In some embodiments, a guide wire may pass through the guidewire lumen 60 of the elongate shaft 24, out of the distal end 28 of the elongate shaft, through the nose cone lumen 74, and out of the distal opening 144 of the nose cone 214. The delivery system 10 may be guided by the guide wire to a location within the patient for installation of the implant 12. In other embodiments, a guide wire is not used, and the elongate shaft 24 may be solid, and not hollow.

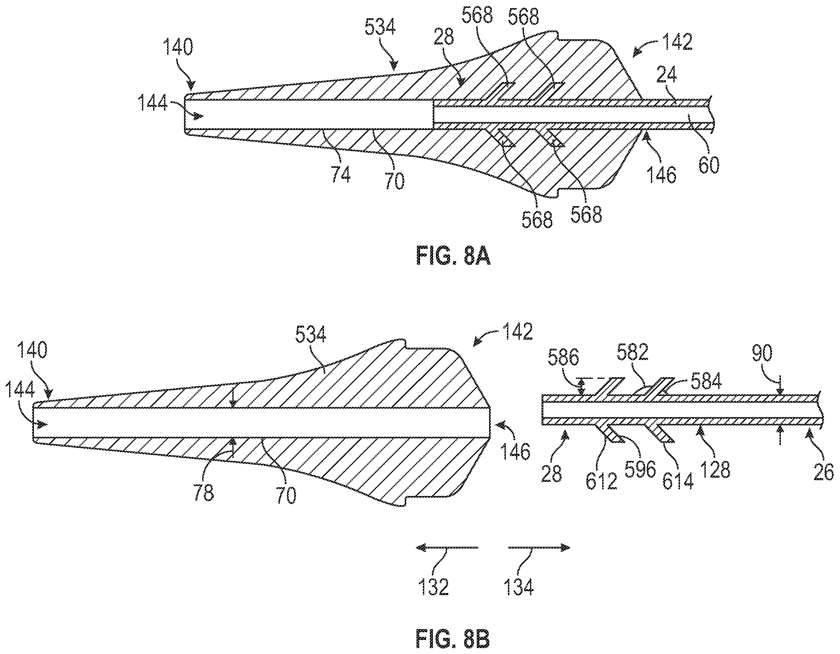

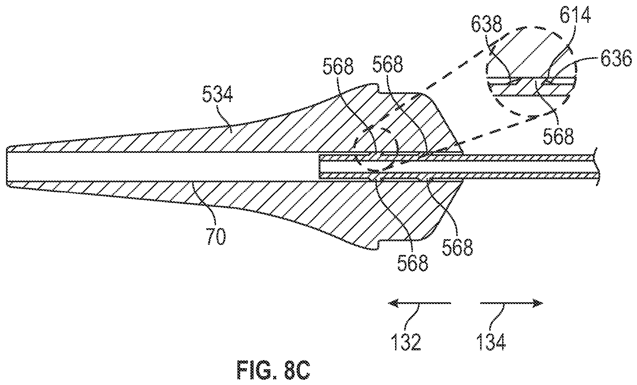

FIGS. 8A-8C illustrate side cross-sectional views along a centerline of a nose cone 534 and an elongate shaft 24. FIG. 8A illustrates the nose cone 534 being coupled to the elongate shaft 24. FIG. 8B illustrates the nose cone 534 and the elongate shaft 24 prior to coupling. FIG. 8C illustrates the nose cone 534 and the elongate shaft 24 during coupling. Nose cone 534 is similar to nose cone 34 with different portions numbered differently and similar portions numbered similarly. The system may include one or more angled projections 568 configured to couple the nose cone 534 to the distal end of the elongate shaft.

As shown in FIG. 8A, the elongate shaft 24 has a guidewire lumen 60 and also has one or more projections 568 extending outward from the elongate shaft 24. In some embodiments, the projections 568 are formed on a sleeve (e.g., a hypotube) that covers and surrounds an outer surface of the elongate shaft 24. The sleeve may be fixedly connected to the elongate shaft 24 such that the sleeve is not capable of axial movement relative to the elongate shaft 24. The sleeve may be connected to the elongate shaft 24 using an adhesive, welding, brazing, or any attachment technique. In other embodiments, the projections 568 are formed integrally on the elongate shaft 24. In these embodiments, portions of the elongate shaft 24 may be cut, and these cut portions may be bent radially outward to form the projections 568. In these embodiments, the projections 568 may be additional structures formed on the outer surface of the elongate shaft 24 using adhesive, welding, brazing, casting, molding, or any other technique.

The nose cone 534 includes a distal opening 144 at a distal end 140 of the nose cone 534 and a proximal opening 146 at a proximal end 142 of the nose cone 534. An inner surface 70 of the nose cone 534 defines a nose cone lumen 74 located between the distal opening 144 and the proximal opening 146. The distal opening 144 may have the same width or a smaller width compared to the proximal opening 146. The proximal opening 146 is configured to receive the distal end 28 of the elongate shaft 24 into the nose cone lumen 74.

The projections 568 of the elongate shaft 24 extend radially outward from the outer surface of the elongate shaft 24 and penetrate and engage the inner surface 70 of the nose cone 534. Accordingly, the projections 568 may have pointed tips that are sufficiently sharp to penetrate the inner surface 70 of the nose cone 534. In this regard, the projections 568 may be referred to as barbs. The projections 568 prevent the elongate shaft 24 from being removed from the proximal opening 146 of the nose cone 534. The projections 568 may be a single annular projection surrounding an outer perimeter of the outer surface of the elongate shaft 24. Alternatively, the projections 568 may be multiple discrete projections spaced around the outer perimeter of the outer surface of the elongate shaft 24. These multiple discrete projections may be spaced evenly apart from each other to evenly distribute any load from the elongate shaft 24. Other configurations may be utilized as desired.

As shown in FIG. 8B, the projections 568 are angled toward the proximal end 26 of the elongate shaft 24. The projections 568 have a proximal side 596 facing the proximal end 142 of the elongate shaft 24 and a distal side 612 facing the distal end 28 of the elongate shaft 24. A distal projection angle 582 is defined by the distal side 612 of the projection 568 and the outer surface 128 of the elongate shaft 24. A proximal projection angle 584 is defined by the proximal side 596 and the outer surface 128 of the elongate shaft 24. The distal projection angle 582 may be an obtuse angle and the proximal projection angle 584 may be an acute angle.

The projections 568 may have a neutral position when no force is exerted upon the projections 568. From the neutral position, the projections 568 are capable of bending or pivoting in the proximal direction 134 to allow the elongate shaft 24 to pass into the nose cone lumen 74. This bending or pivoting of the projections 568 in the proximal direction 134 causes the distal projection angle 582 to be increased relative to the neutral position and the proximal projection angle 584 to be decreased relative to the neutral position. The projections 568 may be biased (e.g., spring-biased) to return to its neutral position after being bent or pivoted in the proximal direction 134. The biasing may be due to the rigidity of the material the elongate shaft 24 is made of. This allows the projections 568 to penetrate and secure the inner surface 70 of the nose cone 534 after the projections 568 have entered the nose cone lumen 74. In contrast, from the neutral position, the projections 568 may be incapable of or resistant to bending or pivoting in the distal direction 132. This inability or resistance allows the projections 568 to retain, secure, and hold the inner surface 70 of the nose cone 534 within the nose cone lumen 74.

The projections 568 have a height 586, which is the distance between the outer surface 128 and the tip 614 of the projections 568. The height 586 of the projections 568 may be optimized to provide retention force onto the nose cone 534 when the elongate shaft 24 exerts force on the projections 568 in the proximal direction 134 and also optimized to allow ease of clearance of the projections 568 past the inner surface 70 of the nose cone 534 when the elongate shaft 24 is being mated with the nose cone 534. Due to the angled orientation of the projections 568, the height 586 is less than the actual length of the projections 568.

The elongate shaft 24 has a width 90 that is less than the width 78 of the nose cone lumen 74, allowing the nose cone lumen 74 to receive the elongate shaft 24.

The elongate shaft 24 is moved in a distal direction 132 (opposite a proximal direction 134) approaching the proximal end 142 of the nose cone 534, to connect the elongate shaft 24 to the nose cone 534. As such, a translation of the proximal end of the nose cone relative to the distal end of the elongate shaft couples the nose cone to the distal end of the elongate shaft.

As shown in FIG. 8C, the nose cone 534 has partially received the elongate shaft 24. The projections 568 are bent or pivoted in the proximal direction 134 to allow the elongate shaft 24 to pass into the nose cone lumen 74. This bending or pivoting of the projections 568 in the proximal direction 134 causes a distal projection angle 638 that is greater than the distal projection angle 582 of the neutral position and also causes a proximal projection angle 636 that is less than the proximal projection angle 584 of the neutral position. Due to the biased nature of the projections 568, the projections 568 press up against the inner surface 70 of the nose cone 534.

Once the projections 568 enter the nose cone lumen 74 and are advanced within the nose cone lumen 74, force may be exerted on the elongate shaft 24 in the proximal direction 134. This force results in the projections 568, which were pressed up against the inner surface 70 of the nose cone 534, to penetrate the inner surface 70 of the nose cone 534. The projections 568 prevent the elongate shaft 24 from being moved further in the proximal direction 134. In some embodiments, when the projections 568 are engaged with the inner surface 70 of the nose cone 534, the projections 568 may be removed from the inner surface 70 of the nose cone 534 by exerting a force onto the elongate shaft 24 in the distal direction 132. In other embodiments, when the projections 568 are engaged with the inner surface 70 of the nose cone 534, the projections 568 may not be removed from the inner surface 70 of the nose cone 534 even if a force is exerted onto the elongate shaft 24 in the distal direction 132.

In some embodiments, a guide wire may pass through the guidewire lumen 60 of the elongate shaft 24, out of the distal end 28 of the elongate shaft, through the nose cone lumen 74, and out of the distal opening 144 of the nose cone 534. The delivery system 10 may be guided by the guide wire to a location within the patient for installation of the implant 12. In other embodiments, a guide wire is not used, and the elongate shaft 24 may be solid, and not hollow.

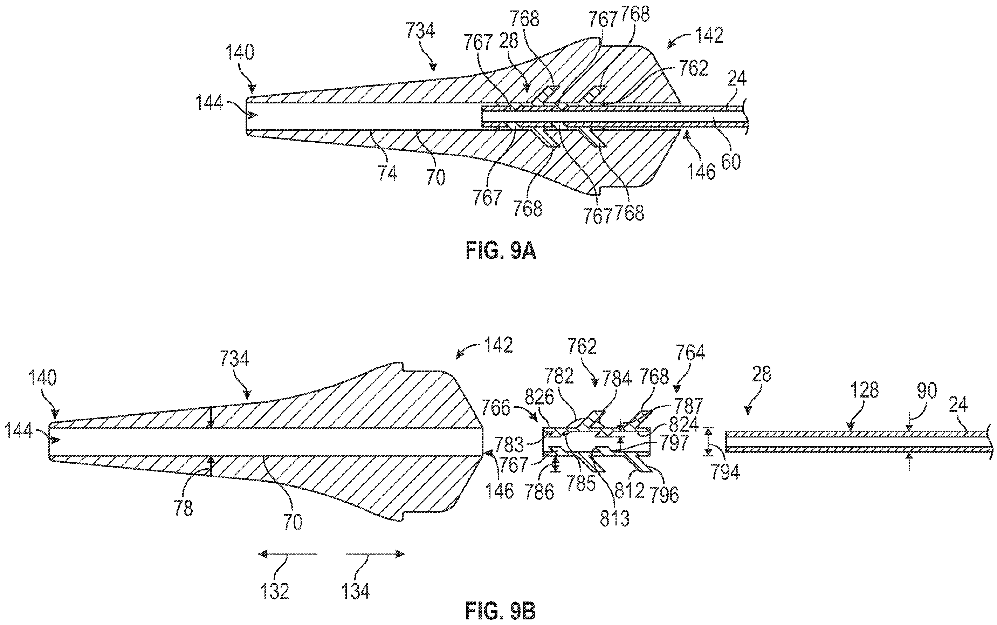

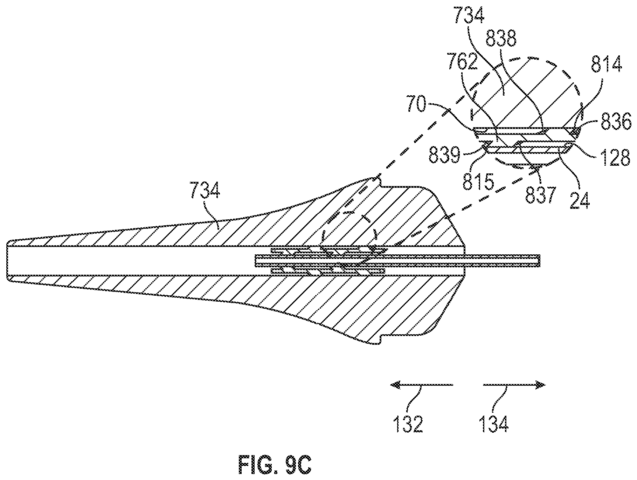

FIGS. 9A-9C illustrate side cross-sectional views along a centerline of a nose cone 734, a retention sleeve 762, and an elongate shaft 24. FIG. 9A illustrates the nose cone 734 coupled to the elongate shaft 24 using the retention sleeve 762. FIG. 9B illustrates the nose cone 734, the retention sleeve 762, and the elongate shaft 24 prior to coupling. FIG. 9C illustrates the nose cone 734, the retention sleeve 762, and the elongate shaft 24 during coupling. Nose cone 734 is similar to nose cone 34 with different portions numbered differently and similar portions numbered similarly. The system may include one or more angled projections 768 configured to couple the nose cone 34 to the distal end of the elongate shaft.

As shown in FIG. 9A, the elongate shaft 24 (having guidewire lumen 60) has a retention sleeve 762 located on a distal portion of the elongate shaft 24. In particular, as shown in FIG. 9B, the retention sleeve 762 surrounds a portion of an outer surface 128 of the elongate shaft 24 such that an inner surface 824 of the retention sleeve 762 contacts the portion of the outer surface 128 of the elongate shaft 24. Referring back to FIG. 9A, the retention sleeve 762 is fixedly connected to the elongate shaft 24 such that the retention sleeve 762 is not capable of axial movement relative to the elongate shaft 24. In some embodiments, the retention sleeve 762 may be a hypotube.

The retention sleeve 762 may include outer projections 768 and inner projections 767. The outer projections 768 of the retention sleeve 762 extend radially outward from the outer surface of the retention sleeve 762 and penetrate and engage the inner surface 70 of the nose cone 734. Accordingly, the projections 768 may have pointed tips that are sufficiently sharp to penetrate the inner surface 70 of the nose cone 734. In this regard, the outer projections 768 may be referred to as barbs. The outer projections 768 prevent the elongate shaft 24 from being removed from the proximal opening 146 of the nose cone 734. The outer projections 768 may be a single annular projection surrounding an outer perimeter of the outer surface of the retention sleeve 762. Alternatively, the outer projections 768 may be multiple discrete projections spaced around the outer perimeter of the outer surface of the retention sleeve 762. These multiple discrete projections may be spaced evenly apart from each other to evenly distribute any load from the elongate shaft 24. In other embodiments, other configurations may be utilized.

The inner projections 767 of the retention sleeve 762 may extend radially inward from the inner surface of the retention sleeve 762 and penetrate and engage the outer surface of the elongate shaft 24. The angle of the inner projections 767 may be in an opposite direction than the outer projections 768. Accordingly, the inner projections 767 may have pointed tips that are sufficiently sharp to penetrate the outer surface of the elongate shaft 24. In this regard, the inner projections 767 may be referred to as barbs. The inner projections 767 prevent the elongate shaft 24 from being decoupled from the retention sleeve 762 and removed from the proximal opening 146 of the nose cone 734. The inner projections 767 may be a single annular projection surrounding an inner perimeter of the inner surface of the retention sleeve 762. Alternatively, the inner projections 767 may be multiple discrete projections spaced around the inner perimeter of the inner surface of the retention sleeve 762. These multiple discrete projections may be spaced evenly apart from each other to evenly distribute any load from the elongate shaft 24.

The outer projections 768 may be formed integrally on the retention sleeve 762. In some embodiments, portions of the retention sleeve 762 are cut, and these cut portions may be bent radially outward to form the outer projections 768. In some embodiments, the outer projections 768 are additional structures formed on the outer surface of the retention sleeve 762 using adhesive, welding, brazing, casting, molding, or any other technique.

The inner projections 767 may be formed integrally on the retention sleeve 762. In some embodiments, portions of the retention sleeve 762 are cut, and these cut portions may be bent radially inward to form the inner projections 767. In some embodiments, the inner projections 767 are additional structures formed on the inner surface of the retention sleeve 762 using adhesive, welding, brazing, casting, molding, or any other technique.

The nose cone 734 includes a distal opening 144 at a distal end 140 of the nose cone 734 and a proximal opening 146 at a proximal end 142 of the nose cone 734. An inner surface 70 of the nose cone 734 defines a nose cone lumen 74 located between the distal opening 144 and the proximal opening 146. The distal opening 144 may have the same width or a smaller width compared to the proximal opening 146. The proximal opening 146 is configured to receive the distal end 28 of the elongate shaft 24 into the nose cone lumen 74.

As shown in FIG. 9B, the retention sleeve 762 has a distal end 766 and a proximal end 764. The outer projections 768 may be angled toward the proximal end 764 of the retention sleeve 762. The outer projections 768 have a proximal side 796 facing the proximal end 764 of the retention sleeve 762 and a distal side 812 facing the distal end 766 of the retention sleeve 762. A distal projection angle 782 is defined by the distal side 812 of the outer projection 768 and the outer surface 826 of the retention sleeve 762. A proximal projection angle 784 is defined by the proximal side 796 and the outer surface 826 of the retention sleeve 762. The distal projection angle 782 may be an obtuse angle and the proximal projection angle 784 may be an acute angle.

The outer projections 768 may have a neutral position when no force is exerted upon the outer projections 768. From the neutral position, the outer projections 768 are capable of bending or pivoting in the proximal direction 134 to allow the retention sleeve 762 to pass into the nose cone lumen 74. This bending or pivoting of the outer projections 768 in the proximal direction 134 causes the distal projection angle 782 to be increased relative to the neutral position and the proximal projection angle 784 to be decreased relative to the neutral position. The outer projections 768 may be biased (e.g., spring-biased) to return to its neutral position after being bent or pivoted in the proximal direction 134. The biasing may be due to the rigidity of the material the retention sleeve 762 is made of. This allows the outer projections 768 to penetrate and secure the inner surface 70 of the nose cone 734 after the outer projections 768 have entered the nose cone lumen 74. In contrast, from the neutral position, the outer projections 768 may be incapable of or resistant to bending or pivoting in the distal direction 132. This inability or resistance allows the outer projections 768 to retain, secure, and hold the inner surface 70 of the nose cone 734 within the nose cone lumen 74.

The outer projections 768 have a height 786, which is the distance between the outer surface 826 and the tip 814 of the outer projections 768. The height 786 of the outer projections 768 may be optimized to provide retention force onto the nose cone 734 when the elongate shaft 24 exerts force on the outer projections 768 in the proximal direction 134 and also optimized to allow ease of clearance of the outer projections 768 past the inner surface 70 of the nose cone 734 when the elongate shaft 24 is being mated with the nose cone 734. Due to the angled orientation of the outer projections 768, the height 786 is less than the actual length of the outer projections 768.

The inner projections 767 may be angled toward the distal end 766 of the retention sleeve 762. The inner projections 767 have a proximal side 797 facing the proximal end 764 of the retention sleeve 762 and a distal side 813 facing the distal end 766 of the retention sleeve 762. A distal projection angle 783 is defined by the distal side 813 of the inner projection 767 and the inner surface 824 of the retention sleeve 762. A proximal projection angle 785 is defined by the proximal side 797 and the inner surface 824 of the retention sleeve 762. The distal projection angle 783 may be an acute angle and the proximal projection angle 785 may be an obtuse angle.

The inner projections 767 may have a neutral position when no force is exerted upon the inner projections 767. From the neutral position, the inner projections 767 are capable of bending or pivoting in the distal direction 132 to allow the elongate shaft 24 to pass into the inner cavity or channel of the retention sleeve 762. This bending or pivoting of the inner projections 767 in the distal direction 132 causes the distal projection angle 783 to be decreased relative to the neutral position and the proximal projection angle 785 to be increased relative to the neutral position. The inner projections 767 may be biased (e.g., spring-biased) to return to its neutral position after being bent or pivoted in the distal direction 132. The biasing may be due to the rigidity of the material the retention sleeve 762 is made of. This allows the inner projections 767 to penetrate and secure the outer surface 128 of the elongate shaft 24 after the elongate shaft 24 has entered the inner cavity or channel of the retention sleeve 762. In contrast, from the neutral position, the inner projections 767 may be incapable of or resistant to bending or pivoting in the proximal direction 134. This inability or resistance allows the inner projections 767 to retain, secure, and hold the outer surface 128 of the elongate shaft 24.

The inner projections 767 have a height 787, which is the distance between the inner surface 824 and the tip 815 of the inner projections 767. The height 787 of the inner projections 767 may be optimized to provide retention force when the elongate shaft 24 exerts force on the inner projections 767 in the proximal direction 134 and also optimized to allow ease of clearance of the inner projections 767 past the outer surface 128 of the elongate shaft 24 when the elongate shaft 24 is being mated with the retention sleeve 762. Due to the angled orientation of the inner projections 767, the height 787 is less than the actual length of the inner projections 767.

The elongate shaft 24 has a width 90 that is less than an inner diameter of the retention sleeve 762, allowing the elongate shaft 24 to pass within the retention sleeve 762. The retention sleeve 762 has an outer diameter 794 that is less than the width 78 of the nose cone lumen 74, allowing the nose cone lumen 74 to receive the elongate shaft 24 and the retention sleeve 762.

The elongate shaft 24 may first be coupled to the retention sleeve 762, and the elongate shaft and retention sleeve combination may then be coupled to the nose cone 734. Alternatively, the retention sleeve 762 may receive the elongate shaft 24 at the proximal end 764 of the retention sleeve 762 at the same time the nose cone 734 receives the retention sleeve 762 at the proximal opening 146.

The elongate shaft 24 and the retention sleeve 762 are moved in a distal direction 132 (opposite a proximal direction 134) approaching the proximal end 142 of the nose cone 734, to connect the elongate shaft 24 to the nose cone 734. As such, a translation of the proximal end of the nose cone relative to the distal end of the elongate shaft couples the nose cone to the distal end of the elongate shaft.

As shown in FIG. 9C, the nose cone 734 has partially received the elongate shaft 24 and the retention sleeve 762. The outer projections 768 are bent or pivoted in the proximal direction 134 to allow the retention sleeve 762 to pass into the nose cone lumen 74. This bending or pivoting of the outer projections 768 in the proximal direction 134 causes a distal projection angle 838 that is greater than the distal projection angle 782 of the neutral position and also causes a proximal projection angle 836 that is less than the proximal projection angle 784 of the neutral position. Due to the biased nature of the outer projections 768, the outer projections 768 press up against the inner surface 70 of the nose cone 734.

The inner projections 767 are bent or pivoted in the distal direction 132 to allow the elongate shaft 24 to be received by the retention sleeve 762. This bending or pivoting of the inner projections 767 in the distal direction 132 causes a distal projection angle 839 that is less than the distal projection angle 783 of the neutral position and also causes a proximal projection angle 837 that is greater than the proximal projection angle 785 of the neutral position. Due to the biased nature of the inner projections 767, the inner projections 767 press up against the outer surface 128 of the elongate shaft 24.

Once the elongate shaft 24 and the retention sleeve 762 enter the nose cone lumen 74 and are advanced within the nose cone lumen 74, force may be exerted on the elongate shaft 24 in the proximal direction 134. This force results in the outer projections 768, which were pressed up against the inner surface 70 of the nose cone 734, to penetrate the inner surface 70 of the nose cone 734. In addition, this force results in the inner projections 767, which were pressed up against the outer surface 128 of the elongate shaft 24, to penetrate the outer surface 128 of the elongate shaft 24. The outer projections 768 and the inner projections 767 prevent the elongate shaft 24 from being moved further in the proximal direction 134.

In some embodiments, a guide wire may pass through the guidewire lumen 60 of the elongate shaft 24, out of the distal end 28 of the elongate shaft, through the nose cone lumen 74, and out of the distal opening 144 of the nose cone 734. The delivery system 10 may be guided by the guide wire to a location within the patient for installation of the implant 12. In other embodiments, a guide wire is not used, and the elongate shaft 24 may be solid, and not hollow.

FIGS. 4A-4D, 5A-5D, 6A-6C, 7A-7D, 8A-8C, and 9A-9C are side cross-sectional views of devices and components that may be annular.

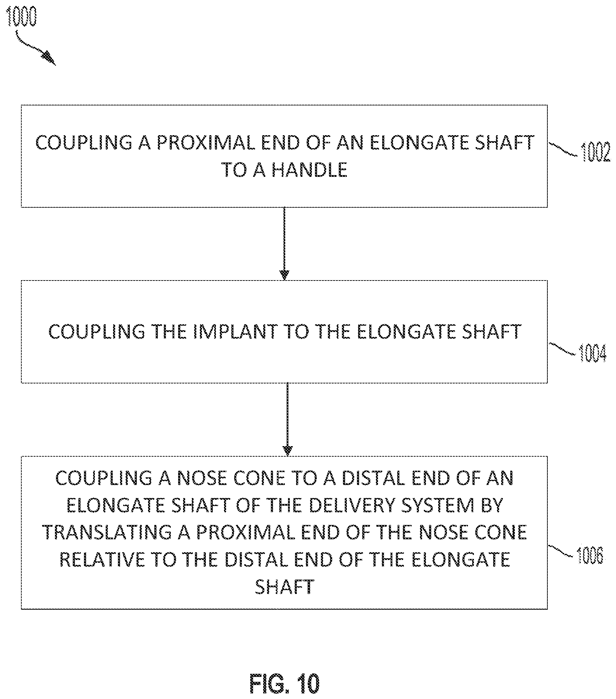

FIG. 10 illustrates a flow chart showing a process 1000 of assembling a delivery system for an implant. In certain embodiments, the proximal end (e.g., the proximal end 26) of an elongate shaft (e.g., elongate shaft 24) may be coupled to a handle (e.g., handle 30) (step 1002). Such a step may occur before, during, or after the remaining steps of the process 1000.

An implant (e.g., implant 12) may be coupled to the elongate shaft (step 1004). In some embodiments, the implant is coupled to an implant retention area (e.g., implant retention area 32) of the elongate shaft. The implant may be covered by a sheath. The implant may also be crimped onto the elongate shaft, as described herein. As compared to conventional methods of assembling delivery systems, the coupling and/or crimping of the implant to the elongate shaft is made easier, more convenient, and more accurate by the absence of the nose cone being rigidly attached to the elongate shaft. As such, the coupling may occur prior to a nose cone being coupled to a distal end of the elongate shaft, and may include crimping the implant to the elongate shaft without the nose cone present. In embodiments, the coupling may occur during or after the remaining steps of the process 1000.

The nose cone (e.g., nose cone 34, 214, 254, 334, 534, 734) may be coupled to the distal end (e.g., distal end 28) of the elongate shaft by translating a proximal end (e.g., proximal end 142) of the nose cone relative to the distal end of the elongate shaft. Examples of such methods are disclosed herein. For example, the translation may include utilizing one or more projections to couple the nose cone to the distal end of the elongate shaft. The translation may include rotating the nose cone about threading to couple the nose cone to the distal end of the elongate shaft. Contrary to methods of molding and forming the nose cone upon the elongate shaft, the nose cone may be pre-formed and then translated relative to the distal end of the elongate shaft. The one or more projections may be engaged to a retention ring (e.g., retention ring 62, 262, 362) located around a portion of the elongate shaft (step 1006). The nose cone may be coupled to the elongate shaft after the implant is coupled to the elongate shaft.

As described herein, one or more projections (e.g., projections 68) located on an inner surface (e.g., inner surface 70) of the nose cone may be engaged by a proximal end (e.g., proximal end 64) of the retention ring when the proximal end of the nose cone is slid over the distal end of the elongate shaft.

Also, as described herein, one or more projections (e.g., projections 368) located on an outer surface (e.g., outer surface 410) of the retention ring may be engaged by a distal end (e.g., distal end 318) of a support ring (e.g.. support ring 302) located within the nose cone when the proximal end of the nose cone is slid over the distal end of the elongate shaft.

The one or more projections are configured to freely bend or pivot in a first direction to allow the nose cone to be coupled to the retention ring. The one or more projections are in a neutral position when no force is applied to the one or more projections, and the one or more projections are configured to prevent bending or pivoting in a second direction past the neutral position.

As described herein, nose cone threads (e.g., nose cone threads 252) may be located on an inner surface of the nose cone and may be engaged by retention ring threads (e.g., retention ring threads 274) located on an outer surface (e.g., outer surface 276) of the retention ring (e.g., retention ring 262) when the proximal end of the nose cone is translated relative to the distal end of the elongate shaft with the elongate shaft or nose cone being rotated about a lengthwise axis (e.g., lengthwise axis 2) of the nose cone.

The use of the nose cones and other components disclosed herein is not limited to use with a delivery system or delivery apparatus, and may extend to use with any medical device to be inserted or withdrawn within a patient's body.

The delivery apparatus and the systems disclosed herein may be used in transcatheter aortic valve implantation (TAVI). The delivery apparatus and the systems disclosed herein may be utilized for transarterial access, including transfemoral access, to a patient's heart. In embodiments, various forms of implants may be delivered by a delivery apparatus utilized with system herein, such as stents or filters, or diagnostic devices, among others.

The delivery apparatuses and the systems and components disclosed herein may be utilized in transcatheter percutaneous procedures, including transarterial procedures, which may be transfemoral or transjugular. Transapical procedures, among others, may also be utilized.

Features of embodiments may be modified, substituted, excluded, or combined.

In addition, the methods herein are not limited to the methods specifically described, and may include methods of utilizing the systems and apparatuses disclosed herein.

The steps of the method may be modified, excluded, or added to, with systems, apparatuses, and methods disclosed herein.

The features of the embodiments disclosed herein may be implemented independently of the delivery apparatuses, or independent of other components disclosed herein. The various apparatuses of the system may be implemented independently.

In closing, it is to be understood that although aspects of the present specification are highlighted by referring to specific embodiments, one skilled in the art will readily appreciate that these disclosed embodiments are only illustrative of the principles of the subject matter disclosed herein. Therefore, it should be understood that the disclosed subject matter is in no way limited to a particular methodology, protocol, and/or reagent, etc., described herein. As such, various modifications or changes to or alternative configurations of the disclosed subject matter can be made in accordance with the teachings herein without departing from the spirit of the present specification. Lastly, the terminology used herein is for the purpose of describing particular embodiments only, and is not intended to limit the scope of systems, apparatuses, and methods as disclosed herein, which is defined solely by the claims. Accordingly, the systems, apparatuses, and methods are not limited to that precisely as shown and described.

Certain embodiments of systems, apparatuses, and methods are described herein, including the best mode known to the inventors for carrying out the same. Of course, variations on these described embodiments will become apparent to those of ordinary skill in the art upon reading the foregoing description. The inventor expects skilled artisans to employ such variations as appropriate, and the inventors intend for the systems, apparatuses, and methods to be practiced otherwise than specifically described herein. Accordingly, the systems, apparatuses, and methods include all modifications and equivalents of the subject matter recited in the claims appended hereto as permitted by applicable law. Moreover, any combination of the above-described embodiments in all possible variations thereof is encompassed by the systems, apparatuses, and methods unless otherwise indicated herein or otherwise clearly contradicted by context.

Groupings of alternative embodiments, elements, or steps of the systems, apparatuses, and methods are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses an approximation that may vary, yet is capable of performing the desired operation or process discussed herein.

The terms "a," "an," "the" and similar referents used in the context of describing the systems, apparatuses, and methods (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the systems, apparatuses, and methods and does not pose a limitation on the scope of the systems, apparatuses, and methods otherwise claimed. No language in the present specification should be construed as indicating any non-claimed element essential to the practice of the systems, apparatuses, and methods.

## Claims

1. A delivery system (10) for an implant, the delivery system comprising:
an elongate shaft (24) having a distal end (28) and a proximal end (26) and including an implant retention area (32) for retaining the implant;
a nose cone (34); and
one or more angled projections (68) configured to couple the nose cone (34) to the distal end (28) of the elongate shaft (24);
wherein:
a.) the one or more angled projections (68) each have a proximal side (96) facing a proximal end (142) of the nose cone (34) and a distal side (112) facing a distal end (140) of the nose cone (34), and
wherein the one or more angled projections (68) are configured to freely bend or pivot toward the distal end (140) of the nose cone (34) when force is applied to the proximal side (96) of the one or more angled projections (68); or
b.) wherein the delivery system further comprises a retention ring (62) coupled to the elongate shaft (24) and having a distal end (66), a proximal end (64), and an outer surface (126), Wherein the one or more angled projections (68, 368) extend outward from the outer surface (126) of the retention ring (62) and are angled toward the proximal end (64) of the retention ring (62), and
wherein the nose cone (34) further comprises a gap (204, 304) located along an inner surface (70) of the nose cone (34) and configured to be engaged by the one or more angled projections (68, 368) to couple the nose cone (34) to the distal end (28) of the elongate shaft.

2. The delivery system of claim 1(a), further comprising a retention ring (62) coupled to the elongate shaft (24), and wherein:
a.) the one or more angled projections (68) are configured to engage the retention ring (62) to couple the nose cone (34) to the distal end (28) of the elongate shaft (24), optionally wherein the one or more angled projections (68) extend inward from an inner surface (70) of the nose cone (34) and are angled toward a distal end (140) of the nose cone (34), the one or more angled projections (68) each having a tip (114) configured to engage a proximal end (64) of the retention ring (62) to couple the nose cone (34) to the distal end (28) of the elongate shaft (24);
b.) the one or more angled projections (68) are formed integrally with the nose cone (34);
c.) the one or more angled projections (68) are connected to a support ring (202) located within a lumen (74) of the nose cone (34) and connected to an inner surface (70) of the nose cone (34); and/or
d.) the one or more angled projections (68) are in a neutral position when no force is applied to the one or more angled projections (68), and the one or more angled projections (68) are configured to prevent bending or pivoting of the one or more angled projections (68) toward the proximal end (142) of the nose cone (34) past the neutral position.

3. The delivery system of claim 1(b), further comprising a support ring (202, 302) located within a lumen (74) of the nose cone (34) and having a proximal end (220, 320), a distal end (218, 318), and an outer surface (208, 308) configured to contact the inner surface (70) of the nose cone (34), the distal end (218, 318) of the support ring (202, 302) forming a surface of the gap (204, 304) of the nose cone (34), and
wherein, optionally:
a.) the one or more angled projections (68, 368) each have a tip (114, 414) configured to engage the distal end (218, 318) of the support ring (202, 302) to couple the nose cone (34) to the distal end (28) of the elongate shaft (24);
b.) the lumen (74) of the nose cone (34) further comprises a support ring portion (216) configured to receive the support ring (202, 302); and/or
c.) the lumen (74) of the nose cone (34) further comprises a lip portion (222, 322) configured to prevent the support ring (202, 302) from being removed from a proximal opening (146) of the nose cone (34).

4. The delivery system of claim 1(b) or claim 3, wherein:
a.) the one or more angled projections (68) each have a proximal side (96, 396) facing the proximal end (64) of the retention ring (62) and a distal side (112, 412) facing the distal end (66) of the retention ring (62), and
wherein the one or more angled projections (68) are configured to freely bend or pivot toward the proximal end (64) of the retention ring (62) when force is applied to the distal side (112, 412) of the one or more angled projections (68); and/or
b.) the one or more angled projections (68) are in a neutral position when no force is applied to the one or more angled projections (68), and the one or more angled projections (68) are configured to prevent bending or pivoting toward the distal end (66) of the retention ring (62) past the neutral position.

5. The delivery system of any of the preceding claims, wherein the nose cone (34) includes
a.) a proximal opening (146) and a distal opening (144), with the proximal opening being larger than the distal opening, and/or
b.) a lumen (74) having a shaft tip portion (120) configured to receive the distal end (28) of the elongate shaft (24), the shaft tip portion (120) having a distal end and a proximal end, optionally further comprising
a retention ring (62) coupled to the elongate shaft (24), and wherein the lumen of the nose cone (34) further comprises a retention ring portion (148) configured to receive the retention ring (62), and having a distal end and a proximal end, the distal end of the retention ring portion (148) being adjacent to the proximal end of the shaft tip portion (120), and further wherein, optionally, the shaft tip portion (120) has a first width (78) and the retention ring portion (148) has a second width (76) greater than the first width (78).

6. The delivery system of claim 1, wherein the one or more angled projections (68) are coupled to a retention sleeve (762) configured to be positioned between the nose cone (34) and the elongate shaft (24),
optionally wherein the retention sleeve (762) includes an outer surface (826) and an inner surface, and the one or more angled projections (68) include one or more angled projections (68, 768) extending from the outer surface (826) of the retention sleeve (762) and one or more angled projections (68, 767) extending from the inner surface of the retention sleeve (762), and further wherein, optionally, the one or more angled projections (68, 762) that extend from the outer surface (826) of the retention sleeve (762) are angled in an opposite direction than the one or more angled projections (68, 767) that extend from the inner surface of the retention sleeve (762).

7. The delivery system of any of the preceding claims, further comprising
a.) a handle (30) positioned at the proximal end (26) of the elongate shaft (24);
b.) a capsule covering the implant retention area (32);
c.) a deflection mechanism (58) configured to cause at least a portion of the elongate shaft (24) to deflect; and/or
d.) a release mechanism configured to release the implant from the implant retention area (32).

8. A method of assembling at least a portion of a delivery system for an implant, the method comprising:
coupling a nose cone (34) to a distal end (28) of an elongate shaft (24) of the delivery system by translating a proximal end (142) of the nose cone (34) relative to the distal end (28) of the elongate shaft (24), the elongate shaft including an implant retention area (32) for retaining the implant,
wherein translating the proximal end (142) of the nose cone (34) includes engaging one or more projections (68) located on an inner surface (70) of the nose cone (34) to a retention ring (62) coupled to the elongate shaft (24), and
wherein the one or more projections (68) are configured to bend or pivot in a first direction to allow the nose cone (34) to be coupled to the retention ring (62).

9. The method of claim 8, wherein
a.) translating the proximal end (142) of the nose cone (34) includes utilizing one or more projections (68) to couple the nose cone to the distal end (28) of the elongate shaft (24), and/or
b.) the one or more projections (68) are in a neutral position when no force is applied to the one or more projections, and the one or more projections are configured to prevent bending or pivoting in a second direction past the neutral position.

10. The method of any of claims 8-9, further comprising coupling (1004) the implant to the elongate shaft (24) prior to the nose cone (34) being coupled to the distal end (24) of the elongate shaft, optionally wherein coupling (1004) the implant to the elongate shaft (24) includes crimping the implant to the elongate shaft.

11. The method of any of claims 8-10, wherein a handle (30) is positioned at a proximal end (26) of the elongate shaft (24).

12. A nose cone (34) for a delivery system for an implant, the nose cone (34) comprising:
a proximal opening (146) at a proximal end (142), the proximal opening configured to receive a distal end (28) of an elongate shaft (24) of the delivery system; and
an inner surface (70) defining a lumen (74), the inner surface (70) configured to engage or having one or more angled projections (68) configured to couple the nose cone (34) to the distal end (28) of the elongate shaft (24);
wherein:
a.) the one or more angled projections (68) each have a proximal side (96) facing the proximal opening (146) of the nose cone (34) and a distal side (112) facing a distal end (140) of the nose cone (34), and
wherein the one or more angled projections (68) are configured to freely bend or pivot toward the distal end (140) of the nose cone (34) when force is applied to the proximal side of the one or more angled projections (68); or
b.) wherein the nose cone (34) further comprises a gap (204, 304) located along the inner surface (70) of the nose cone (34) and configured to be engaged by the one or more angled projections (68).

13. The nose cone of claim 12, wherein:
a.) the one or more angled projections (68) extend inward from the inner surface (70) of the nose cone (34) and are angled toward a distal end (140) of the nose cone;
b.) the one or more angled projections (68) are connected to a support ring (202) located within the lumen (74) of the nose cone (34) and connected to the inner surface (70) of the nose cone (34),
optionally wherein the lumen (74) of the nose cone (34) further comprises a support ring portion (216) configured to receive the support ring (202, 303) and/or a lip portion (222, 322) configured to prevent the support ring (202, 303) from being removed from the proximal opening; and/or
c.) the one or more angled projections (68) are formed integrally with the inner surface (70) of the nose cone (34).

14. The nose cone of claim 12(a), wherein the one or more angled projections (68) are in a neutral position when no force is applied to the one or more angled projections (68), and the one or more angled projections (68) are configured to prevent bending or pivoting toward the proximal end (142) of the nose cone (34) past the neutral position.

15. The nose cone of any of claims 12-14, further comprising a distal opening (144), with the proximal opening (146) being larger than the distal opening (144).

## Patentansprüche

1. Zuführsystem (10) für ein Implantat, wobei das Zuführsystem umfasst:
einen langgestreckten Schaft (24) mit einem distalen Ende (28) und einem proximalen Ende (26), der einen Implantat-Haltebereich (32) zum Halten des Implantats aufweist;
einen Nasenkonus (34); und
einen oder mehrere gewinkelte Vorsprünge (68), die dazu ausgelegt sind, den Nasenkonus (34) mit dem distalen Ende (28) des langgestreckten Schafts (24) zu verbinden;
wobei:
a.) der eine oder die mehreren gewinkelten Vorsprünge (68) jeweils eine proximale Seite (96), die dem proximalen Ende (142) des Nasenkonus (34) zugewandt ist, und eine distale Seite (112), die dem distalen Ende (140) des Nasenkonus (34) zugewandt ist, aufweisen, und
wobei der eine oder die mehreren gewinkelten Vorsprünge (68) dazu ausgelegt sind, sich frei in Richtung des distalen Endes (140) des Nasenkonus (34) zu biegen oder zu verschwenken, wenn auf die proximale Seite (96) des einen oder der mehreren gewinkelten Vorsprünge (68) eine Kraft ausgeübt wird; oder
b.) wobei das Zuführsystem ferner einen Haltering (62) umfasst, der mit dem langgestreckten Schaft (24) verbunden ist und ein distales Ende (66), ein proximales Ende (64), und eine äußere Oberfläche (126) aufweist, wobei sich der eine oder die mehreren gewinkelten Vorsprünge (68, 368) von der äußeren Oberfläche (126) des Halterings (62) nach außen erstrecken und zu dem proximalen Ende (64) des Halteringes (62) hin gewinkelt sind, und
wobei der Nasenkonus (34) ferner einen Spalt (204, 304) umfasst, der entlang einer inneren Oberfläche (70) des Nasenkonus (34) angeordnet und dazu ausgelegt ist, mit dem einen oder den mehreren gewinkelten Vorsprüngen (68, 368) in Eingriff zu gelangen, um den Nasenkonus (34) mit dem distalen Ende (28) des langgestreckten Schafts zu verbinden.

2. Zuführsystem gemäß Anspruch 1(a), ferner umfassend einen Haltering (62), der mit dem langgestreckten Schaft (24) verbunden ist, und wobei:
a.) der eine oder die mehreren gewinkelten Vorsprünge (68) dazu ausgelegt sind, in Eingriff mit dem Haltering (62) zu gelangen, um den Nasenkonus (34) mit dem distalen Ende (28) des langgestreckten Schafts (24) zu verbinden, wobei sich optional der eine oder die mehreren gewinkelten Vorsprünge (68) von einer inneren Oberfläche (70) des Nasenkonus (34) nach innen erstrecken und in Richtung eines distalen Endes (140) des Nasenkonus (34) gewinkelt sind, wobei der eine oder die mehreren gewinkelten Vorsprünge (68) jeweils eine Spitze (114) aufweisen, die dazu ausgelegt ist, in Eingriff mit einem proximalen Ende (64) des Halterings (62) zu gelangen, um den Nasenkonus (34) mit dem distalen Ende (28) des langgestreckten Schafts (24) zu verbinden;
b.) der eine oder die mehreren gewinkelten Vorsprünge (68) integral mit dem Nasenkonus (34) ausgebildet sind;
c.) der eine oder die mehreren gewinkelten Vorsprünge (68) mit einem Stützring (202) verbunden sind, der innerhalb eines Lumens (74) des Nasenkonus (34) angeordnet ist und mit einer inneren Oberfläche (70) des Nasenkonus (34) verbunden ist; und/oder
d.) der eine oder die mehreren gewinkelten Vorsprünge (68) sich in einer neutralen Position befinden, wenn keine Kraft auf den einen oder die mehreren gewinkelten Vorsprünge (68) ausgeübt wird, und der eine oder die mehreren gewinkelten Vorsprünge (68) dazu ausgelegt sind, ein Biegen oder Verschwenken des einen oder der mehreren gewinkelten Vorsprünge (68) hin zu dem proximalen Ende (142) des Nasenkonus (34) über die neutrale Position hinaus zu verhindern.

3. Zuführsystem gemäß Anspruch 1(b), ferner umfassend einen Stützring (202, 302), der innerhalb eines Lumens (74) des Nasenkonus (34) angeordnet ist und ein proximales Ende (220, 320), ein distales Ende (218, 318), und eine äußere Oberfläche (208, 308), die dazu ausgelegt ist, die innere Oberfläche (70) des Nasenkonus (34) zu berühren, aufweist, wobei das distale Ende (218, 318) des Stützringes (202, 302) eine Oberfläche des Spalts (204, 304) des Nasenkonus (34) bildet, und
wobei, optional:
a.) der eine oder die mehreren gewinkelten Vorsprünge (68, 368) jeweils eine Spitze aufweisen (114, 414), die dazu ausgelegt ist, in Eingriff mit dem distalen Ende (218, 318) des Stützringes (202, 302) zu gelangen, um den Nasenkonus (34) mit dem distalen Ende (28) des langgestreckten Schafts (24) zu verbinden;
b.) das Lumen (74) des Nasenkonus (34) ferner einen StützringAbschnitt (216) aufweist, der dazu ausgelegt ist, den Stützring (202, 302) aufzunehmen; und/oder
c.) das Lumen (74) des Nasenkonus (34) ferner einen Lippenabschnitt (222, 322) aufweist, der dazu ausgelegt ist, zu verhindern, dass der Stützring (202, 302) von einer proximalen Öffnung (146) des Nasenkonus (34) entfernt wird.

4. Zuführsystem gemäß Anspruch 1(b) oder Anspruch 3, wobei:
a.) der eine oder die mehreren gewinkelten Vorsprünge (68) jeweils eine proximale Seite (96, 396), die dem proximalen Ende (64) des Halteringes (62) zugewandt ist, und eine distale Seite (112, 412), die dem distalen Ende (66) des Halteringes (62) zugewandt ist, aufweisen, und
wobei der eine oder die mehreren gewinkelten Vorsprünge (68) dazu ausgelegt sind, sich frei in Richtung des proximalen Endes (64) des Halteringes (62) zu biegen oder zu verschwenken, wenn eine Kraft auf die distale Seite (112, 412) des einen oder der mehreren gewinkelten Vorsprünge (68) ausgeübt wird; und/oder
b.) der eine oder die mehreren gewinkelten Vorsprünge (68) sich in einer neutralen Position befinden, wenn keine Kraft auf den einen oder den mehreren gewinkelten Vorsprünge (68) ausgeübt wird, und wobei der eine oder die mehreren gewinkelten Vorsprünge (68) dazu ausgelegt sind, ein Biegen oder Verschwenken in Richtung des distalen Endes (66) des Halteringes (62) über die neutrale Position hinaus zu verhindern.

5. Zuführsystem gemäß einem der vorangehenden Ansprüche, wobei der Nasenkonus (34) aufweist:
a.) eine proximale Öffnung (146) und eine distale Öffnung (144), wobei die proximale Öffnung größer als die distale Öffnung ist, und/oder
b.) ein Lumen (74), das einen Schaftspitzenabschnitt (120) aufweist, der dazu ausgelegt ist, das distale Ende (28 des langgestreckten Schafts (24) aufzunehmen, wobei der Schaftspitzenabschnitt (120) ein distales Ende und ein proximales Ende aufweist, optional ferner umfassend
einen Haltering (62), der mit dem Schaft (24) verbunden ist, und wobei das Lumen des Nasenkonus (34) ferner einen Haltringabschnitt (148) umfasst, der dazu ausgelegt ist, den Haltering (62) aufzunehmen, und der ein distales Ende und ein proximales Ende aufweist, wobei das distale Ende des Halteringabschnittes (148) an das proximale Ende des Schaftspitzenabschnittes (120) angrenzt, und wobei ferner optional der Schaftspitzenabschnitt (120) eine erste Breite (78) aufweist und der Halteringabschnitt (148) eine zweite Breite (76) aufweist, die größer als die erste Breite (78) ist.

6. Zuführsystem gemäß Anspruch 1, wobei der eine oder die mehreren gewinkelten Vorsprünge (68) mit einer Haltehülse (762) verbunden sind, die dazu ausgelegt ist, zwischen dem Nasenkonus (34) und dem langgestreckten Schaft (24) angeordnet zu sein,
wobei optional die Haltehülse (762) eine äußere Oberfläche (826) und eine innere Oberfläche aufweist, und der eine oder die mehreren gewinkelten Vorsprünge (68) einen oder mehrere gewinkelte Vorsprünge (68, 768), der oder die sich von der äußeren Oberfläche (826) der Haltehülse (762) aus erstrecken, und einen oder mehrere gewinkelte Vorsprünge (68, 767), der oder die sich von der inneren Oberfläche der Haltehülse (762) erstrecken, aufweisen, und wobei ferner optional der eine oder die mehreren gewinkelten Vorsprünge (68, 762), die sich von der äußeren Oberfläche (826) der Haltehülse (762) aus erstrecken, in eine entgegengesetzte Richtung gewinkelt sind, als der eine oder die mehreren gewinkelten Vorsprünge (68, 767), die sich von der inneren Oberfläche der Haltehülse (762) erstrecken.

7. Zuführsystem gemäß einem der vorangehenden Ansprüche, ferner umfassend
a.) einen Handgriff (30), der am proximalen Ende (26) des langgestreckten Schafts angeordnet ist;
b.) eine Kapsel, die den Implantatshaltebreich (32) bedeckt;
c.) einen Ablenkmechanismus (58), der dazu ausgelegt ist, mindestens einen Abschnitt des langgestreckten Schafts (24) auszulenken; und/oder
d) einen Freigabemechanismus, der dazu ausgelegt ist, das Implantat aus dem Implantatshaltebereich (32) freizugeben.

8. Verfahren zum Zusammenbauen zumindest eines Teiles eines Zuführsystems für ein Implantat, wobei das Verfahren umfasst:
Verbinden eines Nasenkonus (34) mit einem distalen Ende (28) eines langgestreckten Schafts (24) des Zuführsystems durch Verschieben eines proximalen Endes (142) des Nasenkonus (34) relativ zu dem distalen Ende des langgestreckten Schafts (24), wobei der langgestreckte Schaft einen Implantatshaltebereich (32) zum Halten eines Implantats umfasst,
wobei das Verschieben des proximalen Endes (142) des Nasenkonus (34) beinhaltet, einen oder mehrere Vorsprünge (68), die auf einer inneren Oberfläche (70) des Nasenkonus (34) angeordnet sind, mit dem Haltering (62), der mit dem langgestreckten Schaft (24) verbunden ist, in Eingriff zu bringen, und
wobei der eine oder die mehreren Vorsprünge (68) dazu ausgelegt sind, sich in einer ersten Richtung zu biegen oder zu verschwenken, um dem Nasenkonus (34) zu erlauben, mit dem Haltering (62) verbunden zu werden.

9. Verfahren gemäß Anspruch 8, wobei
a.) das Verschieben des proximalen Endes (142) des Nasenkonus (34) beinhaltet, einen oder mehrere Vorsprünge (68) zu verwenden, um den Nasenkonus mit dem distalen Ende (28) des langgestreckten Schafts (24) zu verbinden, und/oder
b.) der eine oder die mehreren Vorsprünge (68) sich in einer neutralen Position befinden, wenn keine Kraft auf den einen oder die mehreren Vorsprünge ausgeübt wird, und der eine oder die mehreren Vorsprünge dazu ausgelegt sind, ein Biegen oder Verschwenken in eine zweite Richtung über die neutrale Position hinaus zu verhindern.

10. Verfahren gemäß einem der Ansprüche 8 - 9, ferner umfassend Verbinden (1004) des Implantats mit dem langgestreckten Schaft (24), bevor der Nasenkonus (34) mit dem distalen Ende (24) des langgestreckten Schafts verbunden wird, optional wobei das Verbinden (1004) des Implantats mit dem langgestreckten Schaft (24) beinhaltet, das Implantat auf den langgestreckten Schaft zu crimpen.

11. Verfahren gemäß einem der Ansprüche 8 - 10, wobei ein Handgriff (30) an einem proximalen Ende (26) des langgestreckten Schafts (24) angeordnet wird.

12. Nasenkonus (34) für ein Zuführsystem für ein Implantat, wobei der Nasenkonus (34) umfasst:
eine proximale Öffnung (146) an einem proximalen Ende (142), wobei die proximale Öffnung dazu ausgelegt ist, ein distales Ende (28) eines langgestreckten Schafts (24) des Zuführsystems aufzunehmen; und
eine innere Oberfläche (70) ein Lumen bildet, wobei die innere Oberfläche dazu ausgebildet ist, in Eingriff mit einem oder mehreren gewinkelten Vorsprüngen (68), die dazu ausgelegt sind, den Nasenkonus (34) mit dem distalen Ende (28) des langgestreckten Schafts (24) zu verbinden, zu gelangen
wobei:
a.) der eine oder die mehreren gewinkelten Vorsprünge (68) jeweils eine proximale Seite (96), die der proximalen Öffnung (146) des Nasenkonus (34) zugewandt ist, und eine distale Seite (112), die einem distalen Ende (140) des Nasenkonus (34) zugewandt ist, aufweisen, und
wobei der eine oder die mehreren gewinkelten Vorsprünge (68) dazu ausgelegt sind, sich frei in Richtung des distalen Endes (140) des Nasenkonus (34) zu biegen oder zu verschwenken, wenn eine Kraft auf die proximale Seite des einen oder der mehreren gewinkelten Vorsprünge (68) ausgeübt wird, oder
b.) wobei der Nasenkonus (34) ferner einen Spalt (204, 304) umfasst, der entlang der inneren Oberfläche (70) des Nasenkonus (34) angeordnet ist, und der dazu ausgelegt ist, in Eingriff mit dem einen oder den mehreren gewinkelten Vorsprüngen (68) zu gelangen.

13. Nasenkonus gemäß Anspruch 12, wobei:
a.) sich der eine oder die mehreren gewinkelten Vorsprünge (68) von der inneren Oberfläche (70) des Nasenkonus (34) nach innen erstrecken und zu einem distalen Ende (140) des Nasenkonus hin gewinkelt sind;
b.) der eine oder die mehreren gewinkelten Vorsprünge (68) mit einem Stützring (202), der innerhalb des Lumens (74) des Nasenkonus (34) angeordnet ist und mit der inneren Oberfläche (70) des Nasenkonus (34) verbunden ist, verbunden sind,
wobei optional das Lumen (74) des Nasenkonus (34) ferner einen Stützringabschnitt (216), der dazu ausgelegt ist, den Stützring (202, 303) aufzunehmen, und/oder einen Lippenabschnitt (222, 322) aufweist, der dazu ausgelegt ist, zu verhindern, dass der Stützring (202, 302) von der proximalen Öffnung entfernt wird; und/oder
c.) der eine oder die mehreren gewinkelten Vorsprünge (68) einstückig mit der inneren Oberfläche (70) des Nasenkonus (34) ausgestaltet sind.

14. Nasenkonus gemäß Anspruch 12(a), wobei der eine oder die mehreren gewinkelten Vorsprünge (68) sich in einer neutralen Position befinden, wenn keine Kraft auf den einen oder die mehreren gewinkelten Vorsprünge (68) ausgeübt wird, und der eine oder die mehreren gewinkelten Vorsprünge (68) dazu ausgelegt sind, ein Biegen und Verschwenken hin zu dem proximalen Ende (142) des Nasenkonus (34) über die neutrale Position hinaus zu verhindern.

15. Nasenkonus gemäß einem der Ansprüche 12 - 14, ferner umfassend eine distale Öffnung (144), wobei die proximale Öffnung (146) größer ist als die distale Öffnung (144).

## Revendications

1. Système de pose (10) pour un implant, le système de pose comprenant :
une tige allongée (24) présentant une extrémité distale (28) et une extrémité proximale (26) et comprenant une zone de retenue d'implant (32) destinée à retenir l'implant ;
une pointe avant (34) ; et
une ou plusieurs saillies inclinées (68) conçues pour accoupler la pointe avant (34) à l'extrémité distale (28) de la tige allongée (24) ;
a.) ladite une ou lesdites plusieurs saillies inclinées (68) présentant chacune un côté proximal (96) orienté vers une extrémité proximale (142) de la pointe avant (34) et un côté distal (112) orienté vers une extrémité distale (140) de la pointe avant (34), et
ladite une ou lesdites plusieurs saillies inclinées (68) étant conçues pour se courber ou pivoter librement vers l'extrémité distale (140) de la pointe avant (34) lorsqu'une force est appliquée au côté proximal (96) de ladite une ou desdites plusieurs saillies inclinées (68) ; ou
b.) le système de pose comprenant en outre une bague de retenue (62) accouplée à la tige allongée (24) et présentant une extrémité distale (66), une extrémité proximale (64), et une surface externe (126), ladite une ou lesdites plusieurs saillies inclinées (68, 368) s'étendant vers l'extérieur à partir de la surface externe (126) de la bague de retenue (62) et étant inclinées vers l'extrémité proximale (64) de la bague de retenue (62), et
la pointe avant (34) comprenant en outre un interstice (204, 304) situé le long d'une surface interne (70) de la pointe avant (34) et conçu pour être mis en prise par ladite une ou lesdites plusieurs saillies inclinées (68, 368) afin d'accoupler la pointe avant (34) à l'extrémité distale (28) de la tige allongée.

2. Système de pose selon la revendication 1(a), comprenant en outre une bague de retenue (62) accouplée à la tige allongée (24), et :
a.) ladite une ou lesdites plusieurs saillies inclinées (68) étant conçues pour venir en prise avec la bague de retenue (62) afin d'accoupler la pointe avant (34) à l'extrémité distale (28) de la tige allongée (24), éventuellement ladite une ou lesdites plusieurs saillies inclinées (68) s'étendant vers l'intérieur à partir d'une surface interne (70) de la pointe avant (34) et étant inclinées vers une extrémité distale (140) de la pointe avant (34), ladite une ou lesdites plusieurs saillies inclinées (68) présentant chacune une pointe (114) conçue pour venir en prise avec une extrémité proximale (64) de la bague de retenue (62) afin d'accoupler la pointe avant (34) à l'extrémité distale (28) de la tige allongée (24) ;
b.) ladite une ou lesdites plusieurs saillies inclinées (68) étant formées d'un seul tenant avec la pointe avant (34) ;
c.) ladite une ou lesdites plusieurs saillies inclinées (68) étant reliées à une bague support (202) située à l'intérieur d'une lumière (74) de la pointe avant (34) et reliée à une surface interne (70) de la pointe avant (34) ; et/ou
d.) ladite une ou lesdites plusieurs saillies inclinées (68) étant dans une position neutre lorsqu'aucune force n'est appliquée à ladite une ou auxdites plusieurs saillies inclinées (68), et ladite une ou lesdites plusieurs saillies inclinées (68) étant conçues pour empêcher la courbure ou le pivotement de ladite une ou desdites plusieurs saillies inclinées (68) vers l'extrémité proximale (142) de la pointe avant (34) au-delà de la position neutre.

3. Système de pose selon la revendication 1(b), comprenant en outre une bague support (202, 302) située à l'intérieur d'une lumière (74) de la pointe avant (34) et présentant une extrémité proximale (220, 320), une extrémité distale (218, 318) et une surface externe (208, 308) conçue pour entrer en contact avec la surface interne (70) de la pointe avant (34), l'extrémité distale (218, 318) de la bague support (202, 302) formant une surface de l'interstice (204, 304) de la pointe avant (34), et
éventuellement :
a.) ladite une ou lesdites plusieurs saillies inclinées (68, 368) présentant chacune une pointe (114, 414) conçue pour venir en prise avec l'extrémité distale (218, 318) de la bague support (202, 302) afin d'accoupler la pointe avant (34) à l'extrémité distale (28) de la tige allongée (24) ;
b.) la lumière (74) de la pointe avant (34) comprenant en outre une partie (216) à bague support conçue pour recevoir la bague support (202, 302) ; et/ou
c.) la lumière (74) de la pointe avant (34) comprenant en outre une partie lèvre (222, 322) conçue pour empêcher le retrait de la bague support (202, 302) à partir d'une ouverture proximale (146) de la pointe avant (34).

4. Système de pose selon la revendication 1(b) ou la revendication 3 :
a.) ladite une ou lesdites plusieurs saillies inclinées (68) présentant chacune un côté proximal (96, 396) orienté vers l'extrémité proximale (64) de la bague de retenue (62) et un côté distal (112, 412) orienté vers l'extrémité distale (66) de la bague de retenue (62), et
ladite une ou lesdites plusieurs saillies inclinées (68) étant conçues pour se courber ou pivoter librement vers l'extrémité proximale (64) de la bague de retenue (62) lorsqu'une force est appliquée au côté distal (112, 412) de ladite une ou desdites plusieurs saillies inclinées (68) ; et/ou
b.) ladite une ou lesdites plusieurs saillies inclinées (68) étant dans une position neutre lorsqu'aucune force n'est appliquée à ladite une ou auxdites plusieurs saillies inclinées (68) et ladite une ou lesdites plusieurs saillies inclinées (68) étant conçues pour empêcher la courbure ou le pivotement vers l'extrémité distale (66) de la bague de retenue (62) au-delà de la position neutre.

5. Système de pose selon l'une quelconque des revendications précédentes, la pointe avant (34) comprenant
a.) une ouverture proximale (146) et une ouverture distale (144), l'ouverture proximale étant plus grande que l'ouverture distale, et/ou
b.) une lumière (74) présentant une partie (120) à pointe de tige conçue pour recevoir l'extrémité distale (28) de la tige allongée (24), la partie (120) à pointe de tige présentant une extrémité distale et une extrémité proximale, comprenant éventuellement en outre
une bague de retenue (62) accouplée à la tige allongée (24), et la lumière de la pointe avant (34) comprenant en outre une partie (148) à bague de retenue conçue pour recevoir la bague de retenue (62), et présentant une extrémité distale et une extrémité proximale, l'extrémité distale de la partie (148) à bague de retenue étant adjacente à l'extrémité proximale de la partie (120) à pointe de tige et en outre, éventuellement, la partie (120) à pointe de tige présentant une première largeur (78) et la partie (148) à bague de retenue présentant une seconde largeur (76) supérieure à la première largeur (78).

6. Système de pose selon la revendication 1, ladite une ou lesdites plusieurs saillies inclinées (68) étant accouplées à un manchon de retenue (762) conçu pour être positionné entre la pointe avant (34) et la tige allongée (24),
éventuellement le manchon de retenue (762) comprenant une surface externe (826) et une surface interne, et ladite une ou lesdites plusieurs saillies inclinées (68) comprenant une ou plusieurs saillies inclinées (68, 768) s'étendant à partir de la surface externe (826) du manchon de retenue (762) et une ou plusieurs saillies inclinées (68, 767) s'étendant à partir de la surface interne du manchon de retenue (762), et en outre, éventuellement, ladite une ou lesdites plusieurs saillies inclinées (68, 762) qui s'étendent à partir de la surface externe (826) du manchon de retenue (762) étant inclinées dans une direction opposée à celle de ladite une ou desdites plusieurs saillies inclinées (68, 767) qui s'étendent à partir de la surface interne du manchon de retenue (762).

7. Système de pose selon l'une quelconque des revendications précédentes, comprenant en outre
a.) une poignée (30) positionnée au niveau de l'extrémité proximale (26) de la tige allongée (24) ;
b.) une capsule recouvrant la zone de retenue d'implant (32) ;
c.) un mécanisme de déviation (58) conçu pour amener au moins une partie de la tige allongée (24) à dévier ; et/ou
d.) un mécanisme de libération conçu pour libérer l'implant à partir de la zone de retenue d'implant (32).

8. Procédé d'assemblage d'au moins une partie d'un système de pose pour un implant, le procédé comprenant :
l'accouplement d'une pointe avant (34) à une extrémité distale (28) d'une tige allongée (24) du système de pose par translation d'une extrémité proximale (142) de la pointe avant (34) par rapport à l'extrémité distale (28) de la tige allongée (24), la tige allongée comprenant une zone de retenue d'implant (32) destinée à retenir l'implant,
la translation de l'extrémité proximale (142) de la pointe avant (34) comprenant la mise en prise d'une ou de plusieurs saillies (68) situées sur une surface interne (70) de la pointe avant (34) à une bague de retenue (62) accouplée à la tige allongée (24), et
ladite une ou lesdites plusieurs saillies (68) étant conçues pour se courber ou pivoter dans une première direction afin de permettre l'accouplement de la pointe avant (34) à la bague de retenue (62).

9. Procédé selon la revendication 8 :
a.) la translation de l'extrémité proximale (142) de la pointe avant (34) comprenant l'utilisation d'une ou de plusieurs saillies (68) afin d'accoupler la pointe avant à l'extrémité distale (28) de la tige allongée (24), et/ou
b.) ladite une ou lesdites plusieurs saillies (68) étant dans une position neutre lorsqu'aucune force n'est appliquée à ladite une ou auxdites plusieurs saillies et ladite une ou lesdites plusieurs saillies étant conçues pour empêcher la courbure ou le pivotement dans une seconde direction au-delà de la position neutre.

10. Procédé selon l'une quelconque des revendications 8 à 9, comprenant en outre l'accouplement (1004) de l'implant à la tige allongée (24) avant l'accouplement de la pointe avant (34) à l'extrémité distale (24) de la tige allongée, éventuellement l'accouplement (1004) de l'implant à la tige allongée (24) comprenant la compression de l'implant sur la tige allongée.

11. Procédé selon l'une quelconque des revendications 8 à 10, une poignée (30) étant positionnée au niveau d'une extrémité proximale (26) de la tige allongée (24).

12. Pointe avant (34) pour un système de pose pour un implant, la pointe avant (34) comprenant :
une ouverture proximale (146) au niveau d'une extrémité proximale (142), l'ouverture proximale étant conçue pour recevoir une extrémité distale (28) d'une tige allongée (24) du système de pose ; et
une surface interne (70) définissant une lumière (74), la surface interne (70) étant conçue pour venir en prise avec ou présentant une ou plusieurs saillies inclinées (68) conçues pour accoupler la pointe avant (34) à l'extrémité distale (28) de la tige allongée (24) ;
a.) ladite une ou lesdites plusieurs saillies inclinées (68) présentant chacune un côté proximal (96) orienté vers l'extrémité proximale (146) de la pointe avant (34) et un côté distal (112) orienté vers une extrémité distale (140) de la pointe avant (34), et
ladite une ou lesdites plusieurs saillies inclinées (68) étant conçues pour se courber ou pivoter librement vers l'extrémité distale (140) de la pointe avant (34) lorsqu'une force est appliquée au côté proximal de ladite une ou desdites plusieurs saillies inclinées (68) ; ou
b.) la pointe avant (34) comprenant en outre un interstice (204, 304) situé le long de la surface interne (70) de la pointe avant (34) et conçu pour être mis en prise par ladite une ou lesdites plusieurs saillies inclinées (68).

13. Pointe avant selon la revendication 12 :
a.) ladite une ou lesdites plusieurs saillies inclinées (68) s'étendant vers l'intérieur à partir de la surface interne (70) de la pointe avant (34) et étant inclinées vers une extrémité distale (140) de la pointe avant ;
b.) ladite une ou lesdites plusieurs saillies inclinées (68) étant reliées à une bague support (202) située à l'intérieur de la lumière (74) de la pointe avant (34) et reliée à la surface interne (70) de la pointe avant (34),
éventuellement la lumière (74) de la pointe avant (34) comprenant en outre une partie (216) à bague support conçue pour recevoir la bague support (202, 303) et/ou une partie lèvre (222, 322) conçue pour empêcher le retrait de la bague support (202, 303) à partir de l'ouverture proximale ; et/ou
c.) ladite une ou lesdites plusieurs saillies inclinées (68) étant formées d'un seul tenant avec la surface interne (70) de la pointe avant (34).

14. Pointe avant selon la revendication 12(a), ladite une ou lesdites plusieurs saillies inclinées (68) étant dans une position neutre lorsqu'aucune force n'est appliquée à ladite une ou auxdites plusieurs saillies inclinées (68) et ladite une ou lesdites plusieurs saillies inclinées (68) étant conçues pour empêcher la courbure ou le pivotement vers l'extrémité proximale (142) de la pointe avant (34) au-delà de la position neutre.

15. Pointe avant selon l'une quelconque des revendications 12 à 14, comprenant en outre une ouverture distale (144), l'ouverture proximale (146) étant plus grande que l'ouverture distale (144).
